# EUROPEAN PATENT APPLICATION

(11) **EP 4 534 553 A1**
(43) Date of publication of application: **09.04.2025**
(21) Application number: 23811203.1
(22) Date of filing: 29.05.2023
(51) Int. Cl.: C07K 14/575, A61K 38/22, A61P 5/48

(54) **HUMAN AMYLIN ANALOG, AND DERIVATIVE AND USE THEREOF**

(30) Priority: 27.05.2022 CN 202210588910
(71) Applicant: Hangzhou Sciwind Biosciences Co., Ltd., Hangzhou, Zhejiang 310018 (CN); Sciwind Biosciences (Beijing) Co., Ltd., Beijing 100176 (CN)
(72) Inventor: LI, Yan, Beijing 100176 (CN); PAN, Hai, Beijing 100176 (CN); HAO, Sujuan, Beijing 100176 (CN); LI, Zhaoying, Beijing 100176 (CN); LI, Biao, Beijing 100176 (CN); ZOU, Haixia, Beijing 100176 (CN); WU, Xinle, Beijing 100176 (CN)
(74) Representative: dompatent von Kreisler Selting Werner - Partnerschaft von Patent- und Rechtsanwälten mbB
(86) International application number: PCT/CN2023/096811
(87) International publication number: WO 2023/227133

(57) **Abstract**

The present disclosure relates to a polypeptide analog or a derivative thereof, and a preparation method and use thereof, and particularly to an agonistic polypeptide analog or a derivative thereof of an insulin amyloid polypeptide receptor. The polypeptide analog or the derivative thereof provided by the present disclosure has good agonistic activity on the insulin amyloid polypeptide receptor, has the characteristics of good stability, high activity, etc. at the same time, and can be used for treating and preventing diseases related to the insulin amyloid polypeptide receptor, such as body weight abnormality, especially obesity and overweight.

## Description

### TECHNICAL FIELD

The present disclosure relates to the technical field of biomedicine or biopharmaceuticals, and more particularly to a human amylin analog and a derivative thereof, and use thereof for treating and/or preventing a disease related to amylin or metabolism.

### BACKGROUND

Amylin, also known as islet amyloid polypeptide (IAPP), is a member of calcitonin gene-related peptide (CGRP) family, which exerts physiological functions by activating corresponding receptors, and has many functions such as blood glucose regulation, food intake control and metabolism regulation. However, the amylin has a tendency towards fibrosis under neutral or alkaline conditions and is prone to form an amyloid precipitation. Meanwhile, the amylin has a short retention time in blood and a half-life of only about 13 minutes, which limits its use in the treatment of diseases. Therefore, it is of great significance to develop an amylin analog with better stability, longer half-life and more obvious therapeutic effect for the treatment of metabolic diseases such as diabetes and obesity.

Pramlintide (also known as Symlin) is an amylin analog, and by substituting amino acid residues 25, 28 and 29 with Pro, the stability in solution is improved. At present, the Pramlintide has been approved in the market for the treatment of diabetes. However, the half-life of the Pramlintide is only about 48 minutes, so that the efficacy needs to be maintained by frequent injections, and the Pramlintide is prone to aggregate and precipitate under a neutral pH condition, which limits the combined use of the Pramlintide with other therapeutic drugs. WO2012162542A1 discloses a derivative of an amylin polypeptide analog, which is substituted by lysine and linking to polyethylene glycol to prolong the action time of amylin or an analog thereof in vivo, but the affinity of the pegylated derivative to an amylin receptor is obviously decreased. Li-Mei Yan et al. (Proc Natl Acad Sci, 2006,103(7): 2046-2051) disclosed an amylin derivative IAPP-GI, which improved the solubility of amylin by methylation modification of 24-Gly and 26-Ile, but the activity was decreased compared with that of wild-type amylin. Cagrilintide is a long-acting amylin derivative developed by Novo Nordisk Company, which is currently in a phase II clinical stage.

Compared with the amylin, the amylin analogs/derivatives in the prior art have been improved in solubility, stability, half-life, activity, etc., but there are still some technical problems such as insufficient in-vivo efficacy and insufficient drug stability. Therefore, the amylin derivatives still need further improvement.

### SUMMARY

The present disclosure provides a new amylin analog and a derivative thereof, a preparation method thereof, a pharmaceutical composition and product comprising the polypeptide or the derivative thereof, and use thereof in preventing and/or treating a disease.

In one aspect, the present disclosure provides a new amylin analog or a pharmaceutically acceptable salt thereof, wherein the analog comprises an amino acid sequence as shown in X₁CNTA TCATQ RLAX₁₄F LX₁₇HX₁₉X₂₀X₂₁X₂₂X₂₃X₂₄X₂₅ ILX₂₈X₂₉T NVGSX₃₅ TX₃₇ [formula (I)], the amino acid sequence contains one or more amino acid mutations shown in a 1^{st} position, a 14^{th} position, a 17^{th} position, a 19^{th} position, a 20^{th} position, a 21^{st} position, a 22^{nd} position, a 23^{rd} position, a 24^{th} position, a 25^{th} position, a 28^{th} position, a 29^{th} position, a 35^{th} position and a 37^{th} position, and preferably, X₁ is selected from K, D, Dab, Dap, E, H, Har, Orn or R, or deleted, X₁₄ is selected from N, Aad, Dab, Dap, E or D, X₁₇ is selected from V, Orn or R, X₁₉ is selected from S or K, X₂₀ is selected from S or K, X₂₁ is selected from N or K, X₂₂ is selected from N or K, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, X₃₅ is selected from N or G, and X₃₇ is selected from Y, 3-Am-Pro, Aze, cis-Hyp, P, Pip, αMePro or trans-Hyp.

In some preferred embodiments, the amylin analog comprises an amino acid sequence as shown in X₁CNTA TCATQ RLAX₁₄F LX₁₇HSS NNX₂₃X₂₄X₂₅ ILX₂₈X₂₉T NVGSN TX₃₇ [formula (II)], the amino acid sequence contains one or more amino acid mutations shown in a 1^{st} position, a 14^{th} position, a 17^{th} position, a 23^{rd} position, a 24^{th} position, a 25^{th} position, a 28^{th} position, a 29^{th} position and a 37^{th} position, and
preferably, X₁ is selected from K, D, Dab, Dap, E, H, Har, Orn or R, or deleted, X₁₄ is selected from N, Aad, Dab, Dap, E or D, X₁₇ is selected from V, Orn or R, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp;
preferably, X₁ is selected from K or R, X₁₄ is selected from N, Aad, Dab, Dap, E or D, X₁₇ is selected from V, Orn or R, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp;
preferably, X₁ is selected from K, D, Dab, Dap, E, H, Har, Orn or R, or deleted, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp;
preferably, X₁ is selected from K or R, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp;
preferably, X₁ is selected from K, D, Dab, Dap, E, H, Har, Orn or R, or deleted, X₁₄ is selected from N, Aad, Dab, Dap, E or D, X₁₇ is selected from V, Orn or R, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, and X₃₇ is selected from P or trans-Hyp;
preferably, X₁ is selected from K or R, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from P or trans-Hyp;
especially preferably, X₁ is selected from K or R, X₁₄ is selected from N or D, X₁₇ is selected from V or R, X₂₃ is selected from F or L, X₂₄ is selected from G, R or K, X₂₅ is selected from A or P, X₂₈ is selected from S or P, X₂₉ is selected from S or P, and X₃₇ is selected from Y, P or trans-Hyp;
further preferably, X₁ is K, X₁₄ is selected from N or D, X₁₇ is selected from V or R, X₂₃ is selected from F or L, X₂₄ is selected from G, R or K, X₂₅ is selected from A or P, X₂₈ is selected from S or P, X₂₉ is selected from S or P, and X₃₇ is selected from Y, P or trans-Hyp;
further preferably, X₁ is R, X₁₄ is selected from N or D, X₁₇ is selected from V or R, X₂₃ is selected from F or L, X₂₄ is selected from G, R or K, X₂₅ is selected from A or P, X₂₈ is selected from S or P, X₂₉ is selected from S or P, and X₃₇ is selected from Y, P or trans-Hyp;
further preferably, X₁ is selected from K or R, X₁₄ is D, X₁₇ is R, X₂₃ is L, X₂₅ is P, X₂₄ is selected from G, R or K, X₂₈ is P, X₂₉ is P, and X₃₇ is P or trans-Hyp;
more preferably, X₁ is K, X₁₄ is D, X₁₇ is R, X₂₃ is L, X₂₄ is selected from G or K, X₂₅ is P, X₂₈ is P, X₂₉ is P, and X₃₇ is P or trans-Hyp; and
most preferably, X₁ is K, X₁₄ is D, X₁₇ is R, X₂₃ is L, X₂₄ is K, X₂₅ is P, X₂₈ is P, X₂₉ is P, and X₃₇ is P or trans-Hyp.

In one aspect, the present disclosure provides a new amylin analog derivative or a pharmaceutically acceptable salt thereof, wherein the amylin analog derivative has one or more amino acid modifications, and the analog derivative comprises a polypeptide sequence of the amylin analog mentioned above.

In some embodiments, the polypeptide analog or the derivative thereof in the present disclosure comprises an amino acid sequence as shown in SEQ ID NO. 2 to 104 in Table 1.

**Table 1**

| SEQ ID NO. | Amino acids |
|---|---|
| 1 | KCNTATCATQRLAEFLRHSSNNFGPILPPTNVGSNTP-NH₂ |
| 2 | KCNTATCATQRLADFLRHSSNNLGPILPPTNVGSNT-trans-Hyp-NH₂ |
| 3 | KCNTATCATQRLADFLRHSSNNLKPILPPTNVGSNT-trans-Hyp-NH₂ |
| 4 | RCNTATCATQRLADFLRHSKNNLGPILPPTNVGSNT-trans-Hyp-NH₂ |
| 5 | RCNTATCATQRLAEFLRHSSKNLGPILPPTNVGSNTP-NH₂ |
| 6 | RCNTATCATQRLAEFLRHSSNKLGPILPPTNVGSNTP-NH₂ |
| 7 | RCNTATCATQRLAEFLRHSSNNKGPILPPTNVGSNTP-NH₂ |
| 8 | RCNTATCATQRLAEFLRHSSNNLKPILPPTNVGSNTP-NH₂ |
| 9 | RCNTATCATQRLAEFLRHSSNNLGKILPPTNVGSNTP-NH₂ |
| 10 | RCNTATCATQRLAEFLRHSSNNLKPILSSTNVGSNTP-NH₂ |
| 11 | RCNTATCATQRLAEFLRHSSNNLKAILPSTNVGSNTP-NH₂ |
| 12 | RCNTATCATQRLAEFLRHSSNNLKAILSPTNVGSNTP-NH₂ |
| 13 | RCNTATCATQRLAEFLRHSSNNLKPILPSTNVGSNTP-NH₂ |
| 14 | RCNTATCATQRLAEFLRHSSNNLKPILSPTNVGSNTP-NH₂ |
| 15 | RCNTATCATQRLAEFLRHSSNNLKAILPPTNVGSNTP-NH₂ |
| 16 | RCNTATCATQRLAEFLRHSSNNLKPILPPTNVGSNTY-NH₂ |
| 17 | RCNTATCATQRLAEFLRHSSNNLKAILSSTNVGSNTY-NH₂ |
| 18 | RCNTATCATQRLAEFLRHSSNNLKPILSSTNVGSNTY-NH₂ |
| 19 | RCNTATCATQRLAEFLRHSSNNLKAILPSTNVGSNTY-NH₂ |
| 20 | RCNTATCATQRLAEFLRHSSNNLKAILSPTNVGSNTY-NH₂ |
| 21 | RCNTATCATQRLAEFLRHSSNNLKPILPSTNVGSNTY-NH₂ |
| 22 | RCNTATCATQRLAEFLRHSSNNLKPILSPTNVGSNTY-NH₂ |
| 23 | RCNTATCATQRLAEFLRHSSNNLKAILPPTNVGSNTY-NH₂ |
| 24 | RCNTATCATQRLAEFLRHSSNNLKPILPPTNVGSGTP-NH₂ |
| 25 | RCNTATCATQRLAEFLRHSSNNLKAILSSTNVGSGTP-NH₂ |
| 26 | RCNTATCATQRLAEFLRHSSNNLKPILSSTNVGSGTP-NH₂ |
| 27 | RCNTATCATQRLAEFLRHSSNNLKAILPSTNVGSGTP-NH₂ |
| 28 | RCNTATCATQRLAEFLRHSSNNLKAILSPTNVGSGTP-NH₂ |
| 29 | RCNTATCATQRLAEFLRHSSNNLKPILPSTNVGSGTP-NH₂ |
| 30 | RCNTATCATQRLAEFLRHSSNNLKPILSPTNVGSGTP-NH₂ |
| 31 | RCNTATCATQRLAEFLRHSSNNLKAILPPTNVGSGTP-NH₂ |
| 32 | KCNTATCATQRLAEFLRHSSNNLKPILPPTNVGSNTP-NH₂ |
| 33 | KCNTATCATQRLAEFLRHSSNNLKPILSSTNVGSNTP-NH₂ |
| 34 | KCNTATCATQRLAEFLRHSSNNLKAILPSTNVGSNTP-NH₂ |
| 35 | KCNTATCATQRLAEFLRHSSNNLKAILSPTNVGSNTP-NH₂ |
| 36 | KCNTATCATQRLAEFLRHSSNNLKPILPSTNVGSNTP-NH₂ |
| 37 | KCNTATCATQRLAEFLRHSSNNLKPILSPTNVGSNTP-NH₂ |
| 38 | KCNTATCATQRLAEFLRHSSNNLKAILPPTNVGSNTP-NH₂ |
| 39 | RCNTATCATQRLAEFLRHSSNNLKPILPPTNVGSNT-trans-Hyp-NH₂ |
| 40 | RCNTATCATQRLAEFLRHSSNNLKPILPPTNVGSGT-trans-Hyp-NH₂ |
| 41 | RCNTATCATQRLADFLRHSSNNLKPILPPTNVGSNTP-NH₂ |
| 42 | RCNTATCATQRLA-Aad-FLRHSSNNLKPILPPTNVGSNTP-NH₂ |
| 43 | RCNTATCATQRLA-Dab-FLRHSSNNLKPILPPTNVGSNTP-NH₂ |
| 44 | RCNTATCATQRLA-Dap-FLRHSSNNLKPILPPTNVGSNTP-NH₂ |
| 45 | RCNTATCATQRLAEFL-Orn-HSSNNLKPILPPTNVGSNTP-NH₂ |
| 46 | RCNTATCATQRLAEFLRHSSNNLKPILPPTNVGSNTP-NH₂ |
| 47 | RCNTATCATQRLADFLRHSSNNLKPILPPTNVGSNTP-NH₂ |
| 48 | CNTATCATQRLAEFLRHSSNNLKPILPPTNVGSNTP-NH₂ |
| 49 | CNTATCATQRLADFLRHSSNNLKPILPPTNVGSNTP-NH₂ |
| 50 | RCNTATCATQRLADFLRHSSNNLKPILPPTNVGSNT-trans-Hyp-NH₂ |
| 51 | CNTATCATQRLAEFLRHSSNNLKPILPPTNVGSNT-trans-Hyp-NH₂ |
| 52 | CNTATCATQRLADFLRHSSNNLKPILPPTNVGSNT-trans-Hyp-NH₂ |
| 53 | RCNTATCATQRLAEFLRHSSNNLKPILPPTNVGSNT-cis-Hyp-NH₂ |
| 54 | RCNTATCATQRLADFLRHSSNNLKPILPPTNVGSNT-cis-Hyp-NH₂ |
| 55 | RCNTATCATQRLAEFLRHSSNNLKPILPPTNVGSNT-Pip-NH₂ |
| 56 | RCNTATCATQRLADFLRHSSNNLKPILPPTNVGSNT-Pip-NH₂ |
| 57 | RCNTATCATQRLAEFLRHSSNNLKPILPPTNVGSNT-Aze-NH₂ |
| 58 | RCNTATCATQRLADFLRHSSNNLKPILPPTNVGSNT-Aze-NH₂ |
| 59 | RCNTATCATQRLAEFLRHSSNNLKPILPPTNVGSNT-3-Am-Pro-NH₂ |
| 60 | RCNTATCATQRLADFLRHSSNNLKPILPPTNVGSNT-3-Am-Pro-NH₂ |
| 61 | RCNTATCATQRLAEFLRHSSNNLKPILPPTNVGSNT-αMePro-NH₂ |
| 62 | RCNTATCATQRLADFLRHSSNNLKPILPPTNVGSNT-αMePro-NH₂ |
| 63 | KCNTATCATQRLADFLRHSSNNFGPILPPTNVGSNT-trans-Hyp-NH₂ |
| 64 | Dap-CNTATCATQRLADFLRHSSNNLKPILPPTNVGSNT-trans-Hyp-NH₂ |
| 65 | Dab-CNTATCATQRLADFLRHSSNNLKPILPPTNVGSNT-trans-Hyp-NH₂ |
| 66 | Orn-CNTATCATQRLADFLRHSSNNLKPILPPTNVGSNT-trans-Hyp-NH₂ |
| 67 | Har-CNTATCATQRLADFLRHSSNNLKPILPPTNVGSNT-trans-Hyp-NH₂ |
| 68 | HCNTATCATQRLADFLRHSSNNLKPILPPTNVGSNT-trans-Hyp-NH₂ |
| 69 | DCNTATCATQRLADFLRHSSNNLKPILPPTNVGSNT-trans-Hyp-NH₂ |
| 70 | ECNTATCATQRLADFLRHSSNNLKPILPPTNVGSNT-trans-Hyp-NH₂ |
| 71 | RCNTATCATQRLADFLRHSSNNKGPILPPTNVGSNT-trans-Hyp-NH₂ |
| 72 | RCNTATCATQRLADFLRHSSNKLGPILPPTNVGSNT-trans-Hyp-NH₂ |
| 73 | RCNTATCATQRLADFLRHSSKNLGPILPPTNVGSNT-trans-Hyp-NH₂ |
| 74 | RCNTATCATQRLADFLRHKSNNLGPILPPTNVGSNT-trans-Hyp-NH₂ |
| 75 | RCNTATCATQRLADFLRHSSNNLGKILPPTNVGSNT-trans-Hyp-NH₂ |
| 76 | RCNTATCATQRLADFLRHSSNNLGPILPPTNVGSNT-trans-Hyp-NH₂ |
| 77 | HCNTATCATQRLADFLRHSSNNLGPILPPTNVGSNT-trans-Hyp-NH₂ |
| 78 | KCNTATCATQRLANFLVHSSNNFGPILPPTNVGSNTY |
| 79 | KCNTATCATQ RLADFLRHSS NNLKPILPPT NVGSNT-Aze-NH₂ |
| 80 | KCNTATCATQ RLADFLRHSS NNLKPILPPT NVGSNT-Aze-NH₂ |
| 81 | KCNTATCATQ RLANFLVHSS NNFGPILPPT NVGSNTY-NH₂ |
| 82 | KCNTATCATQ RLADFLRHSS NNLKPILPPT NVGSNT-Aze-NH₂ |
| 83 | KCNTATCATQ RLADFLRHSS NNLRPILPPT NVGSNT-trans-Hyp-NH₂ |
| 84 | KCNTATCATQ RLADFLRHSS NNLHPILPPT NVGSNT-trans-Hyp-NH₂ |
| 85 | KCNTATCATQ RLADFLRHSS NNLKPILPPT NVGSNTP-NH₂ |
| 86 | KCNTATCATQ RLADFLRHSS NNLRPILPPT NVGSNTP-NH₂ |
| 87 | KCNTATCATQ RLADFLRHSS NNLHPILPPT NVGSNTP-NH₂ |
| 88 | KCNTATCATQ RLADFLRHSS NNLPPILPPT NVGSNTP-NH₂ |
| 89 | KCNTATCATQ RLADFLRHSS NNLDPILPPT NVGSNTP-NH₂ |
| 90 | KCNTATCATQ RLADFLRHSS NNLNPILPPT NVGSNTP-NH₂ |
| 91 | KCNTATCATQ RLADFLRHSS NNLSPILPPT NVGSNTP-NH₂ |
| 92 | KCNTATCATQ RLADFLRHSS NNL-αK-PILPPT NVGSNTP-NH₂ |
| 93 | KCNTATCATQ RLADFLRHSS NNL-Dap-PILPPT NVGSNTP-NH₂ |
| 94 | KCNTATCATQ RLADFLRHSS NNL-Dab-PILPPT NVGSNTP-NH₂ |
| 95 | KCNTATCATQ RLADFLRHSS NNL-Orn-PILPPT NVGSNTP-NH₂ |
| 96 | KCNTATCATQ RLADFLRHSS NNL-Cit-PILPPT NVGSNTP-NH₂ |
| 97 | KCNTATCATQ RLADFLRHSS NNL-Sar-PILPPT NVGSNTP-NH₂ |
| 98 | KCNTATCATQ RLADFLRHSS NNL-Trx-PILPPT NVGSNTP-NH₂ |
| 99 | KCNTATCATQ RLADFLRHSS NNL-AOC-PILPPT NVGSNTP-NH₂ |
| 100 | KCNTATCATQ RLADFLRHSS NNLGPILPPT NVGSNT-Aze-NH₂ |
| 101 | KCNTATCATQ RLADFLRHSS NNLKPILPPT NVGSNT-trans-Hyp-NH₂ |
| 102 | KCNTATCATQ RLADFLRHSS NNLKPILPPT NVGSNTP-NH₂ |
| 103 | RCNTA TCATQ RLADF LRHSS NNLRP ILPPT NVGSN TP-NH₂ |
| 104 | KGGGG RCNTA TCATQ RLADF LRHSS NNLRP ILPPT NVGSN TP-NH₂ |

In some embodiments, compared with a sequence of a wild-type polypeptide, the sequence of the polypeptide analog derivative in the present disclosure comprises 1 to 10 amino acid modifications, preferably 1 to 8 amino acid modifications, further preferably 1 to 4 amino acid modifications, more preferably 2 amino acid modifications, and most preferably 1 amino acid modification.

In some embodiments, compared with the sequence of the wild-type polypeptide, the sequence of the polypeptide analog derivative is subjected to amino acid modification(s) in which a functional group capable of being used for coupling is introduced into a side chain of natural amino acid or unnatural amino acid, which comprises, but is not limited to, glutamic acid, aspartic acid, lysine, glutamine or asparagine, preferably, the amino acid modification is carried out on the side chain of lysine, preferably, the lysine is located in a first position of the polypeptide derivative, and the modified side chain is selected from an Alpha-amino or Epsilon-amino side chain of lysine, preferably the Epsilon-amino side chain.

In some embodiments, the functional group capable of being used for coupling comprises an extension part and a linker linking the extension part to a modified amino acid, wherein the linker may be composed of one or more linkers, and a linking order of the linkers is arbitrary, as long as the linkage of the extension part to the modified amino acid can be understood as being in a form within the scope of the present disclosure.

In some preferred embodiments, the single linker may be in the following structure: 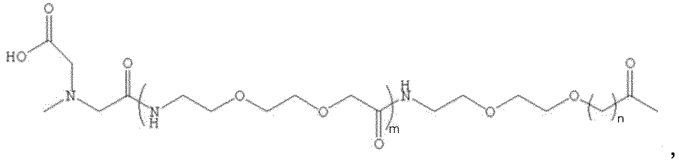 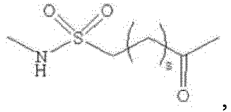 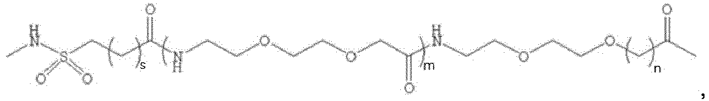 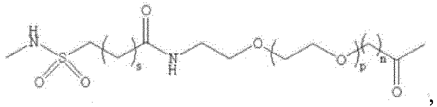 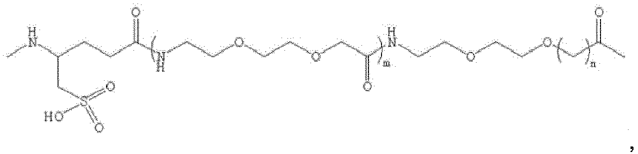 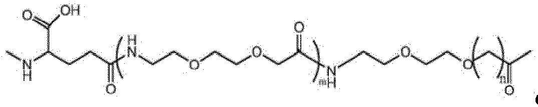 or 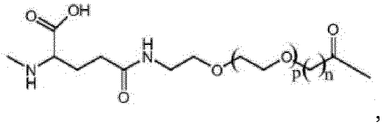 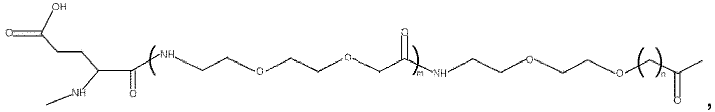 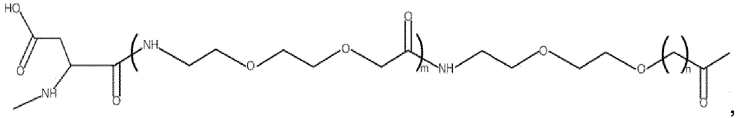 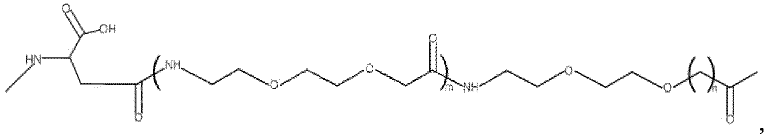 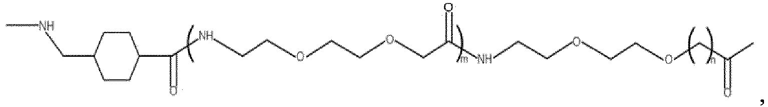 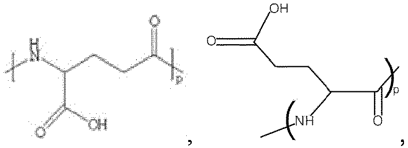 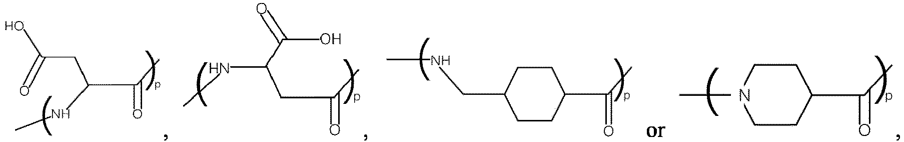 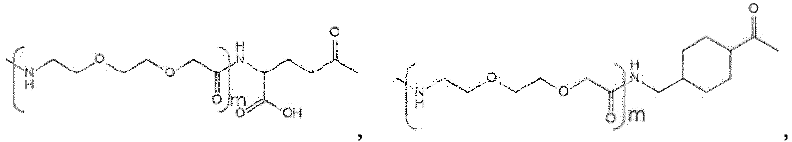 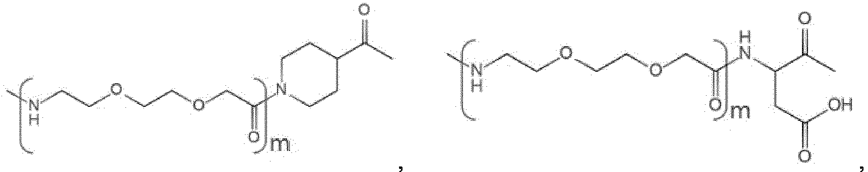 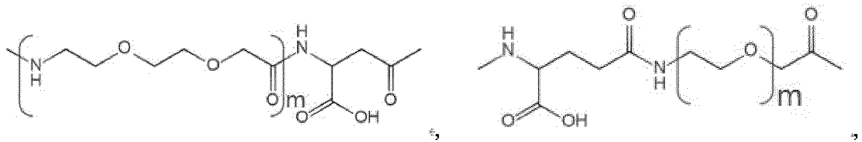 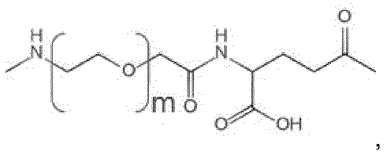 wherein, m is 0, 1, 2 or 3; n is 1, 2 or 3; s is any integer from 0 to 6; and p is any integer from 1 to 8.

Preferably, the linker is: 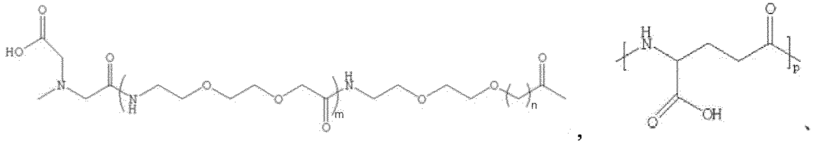 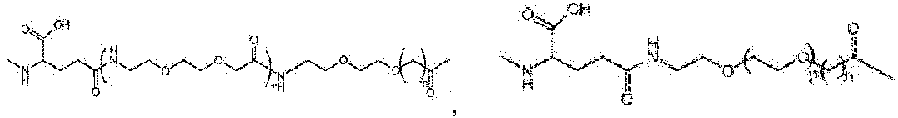 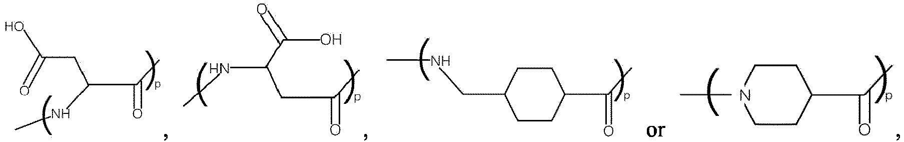 wherein, m is 1 or 2; n is 1 or 2; and p is any integer from 1 to 5.

Further preferably, the linker is: 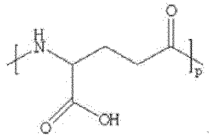
wherein, p is any integer from 1 to 5,
preferably, p is 1, and the linker is γGlu.

In preferred embodiments, the extension part is: 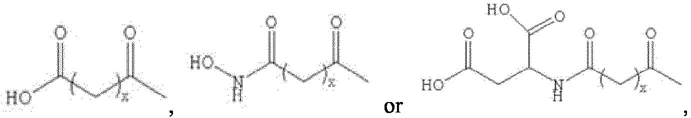
wherein, x is any integer from 4 to 38; and
preferably, the extension part is 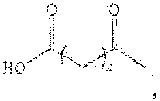 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is selected from K or R, X₁₄ is selected from N, Aad, Dab, Dap, E or D, X₁₇ is selected from V, Orn or R, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp; and the linker is: 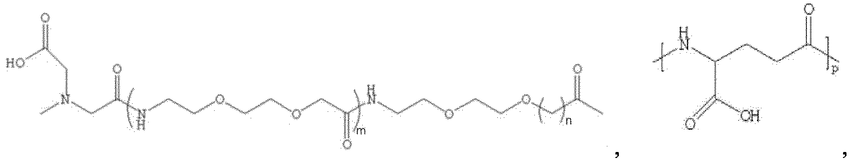 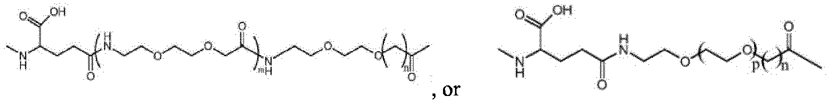
wherein m is 1 or 2; n is 1 or 2; p is any integer from 1 to 5; and the extension part is 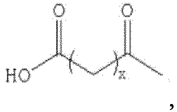 wherein x is any integer from 4 to 38.

Preferably, the amino acid sequence of the polypeptide analog comprises the amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is selected from K, D, Dab, Dap, E, H, Har, Orn or R, or deleted, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp; and the linker is: 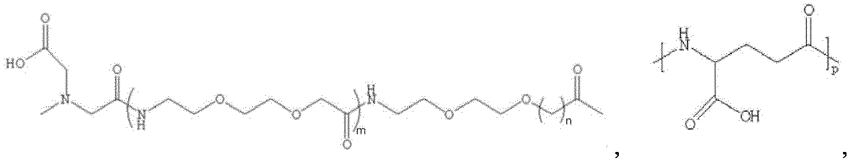 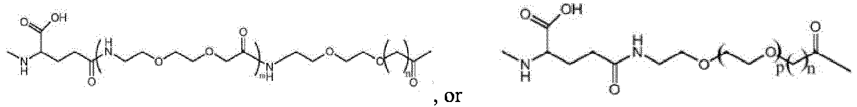
wherein m is 1 or 2; n is 1 or 2; p is any integer from 1 to 5; and the extension part is 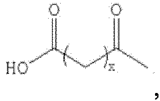 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),

wherein, X₁ is selected from K or R, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from Y, 3-Am-Pro, Aze, cis-Hyp, P, Pip, αMePro or trans-Hyp; and particularly preferably, X₁ is selected from K or R, X₁₄ is selected from N or D, X₁₇ is selected from V or R, X₂₃ is selected from F or L, X₂₄ is selected from G or K, X₂₅ is selected from A or P, X₂₈ is selected from S or P, X₂₉ is selected from S or P, and X₃₇ is selected from Y or trans-Hyp; and the linker is: 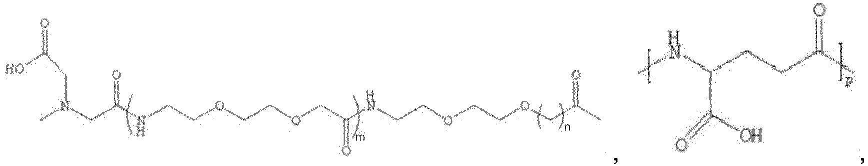 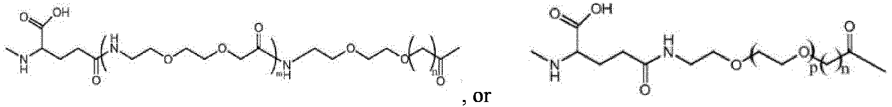
wherein m is 1 or 2; n is 1 or 2; p is any integer from 1 to 5; and the extension part is 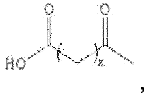 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is K, X₁₄ is selected from N or D, X₁₇ is selected from V or R, X₂₃ is selected from F or L, X₂₄ is selected from G or K, X₂₅ is selected from A or P, X₂₈ is selected from S or P, X₂₉ is selected from S or P, and X₃₇ is selected from Y or trans-Hyp; and the linker is: 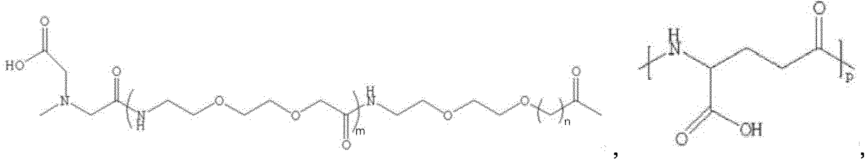 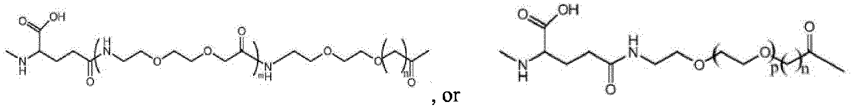
wherein m is 1 or 2; n is 1 or 2; p is any integer from 1 to 5; and the extension part is 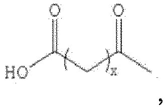 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),

wherein, X₁ is R, X₁₄ is selected from N or D, X₁₇ is selected from V or R, X₂₃ is selected from F or L, X₂₄ is selected from G or K, X₂₅ is selected from A or P, X₂₈ is selected from S or P, X₂₉ is selected from S or P, and X₃₇ is selected from Y or trans-Hyp; and the linker is: 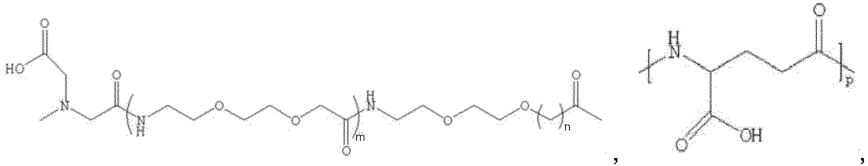 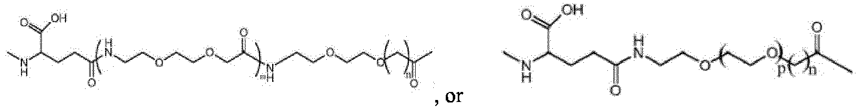
wherein m is 1 or 2; n is 1 or 2; p is any integer from 1 to 5; and the extension part is 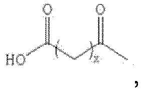 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is selected from K or R, X₁₄ is D, X₁₇ is R, X₂₃ is L, X₂₅ is P, X₂₄ is selected from G or K, X₂₈ is P, X₂₉ is P, and X₃₇ is trans-Hyp; and the linker is: 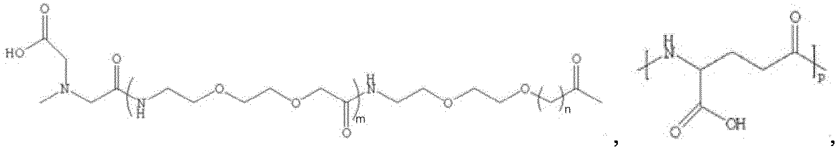 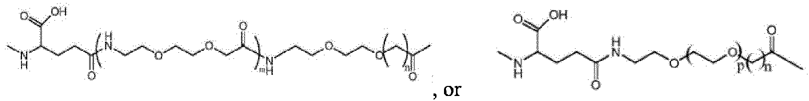
wherein m is 1 or 2; n is 1 or 2; p is any integer from 1 to 5; and the extension part is 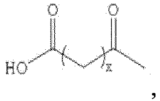 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is K, X₁₄ is D, X₁₇ is R, X₂₃ is L, X₂₄ is selected from G or K, X₂₅ is P, X₂₈ is P, X₂₉ is P, and X₃₇ is trans-Hyp; and the linker is: 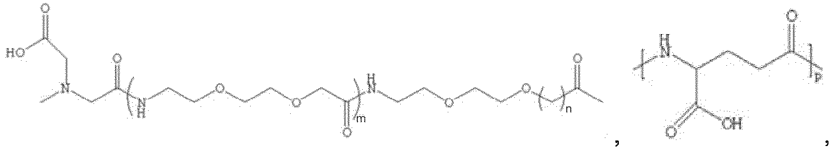 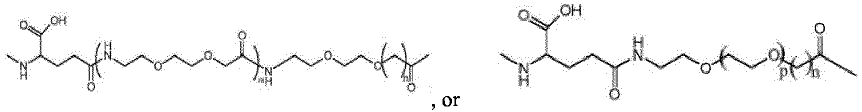
wherein m is 1 or 2; n is 1 or 2; p is any integer from 1 to 5; and the extension part is 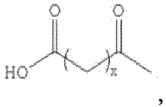 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein, X₁ is K, X₁₄ is D, X₁₇ is R, X₂₃ is L, X₂₄ is K, X₂₅ is P, X₂₈ is P, X₂₉ is P, and X₃₇ is trans-Hyp; and the linker is: 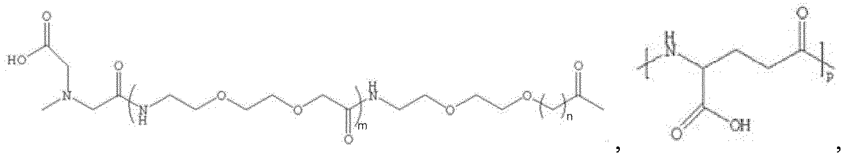 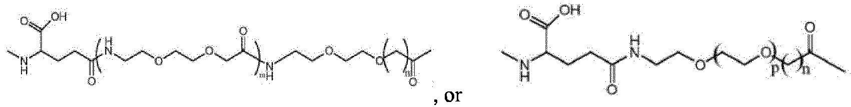
wherein m is 1 or 2; n is 1 or 2; p is any integer from 1 to 5; and the extension part is 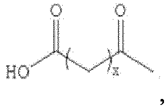 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),

X₁ is selected from K, D, Dab, Dap, E, H, Har, Orn or R, or deleted, X₁₄ is selected from N, Aad, Dab, Dap, E or D, X₁₇ is selected from V, Orn or R, X₂₃ is selected from F, L or K, X₂₄ is selected from G, R, H, P, D, N, S, K, αK, Dap, Dab, Orn, Cit, Sar, Trx or AOC, X₂₅ is selected from A, K or P, X₂₈ is selected from S or P, X₂₉ is selected from S or P, and X₃₇ is selected from P or trans-Hyp; and the linker is: 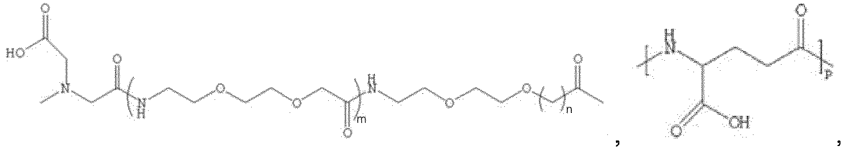 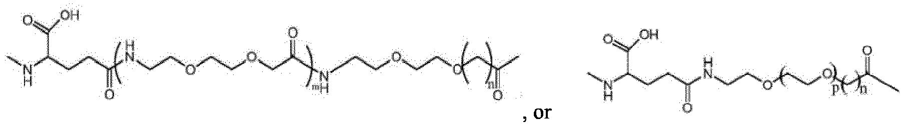
wherein m is 1 or 2; n is 1 or 2; p is any integer from 1 to 5; and the extension part is 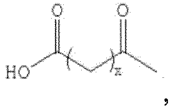 wherein x is any integer from 4 to 38.

Preferably, the amino acid sequence of the polypeptide analog comprises an amino acid sequence as shown in formula (I):

X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),

X₁ is selected from K or R, X₁₄ is selected from N, Aad, Dab, Dap, E or D, X₁₇ is selected from V, Orn or R, X₂₃ is selected from F, L or K, X₂₄ is selected from G, R or K, X₂₅ is selected from A, K or P, X₂₈ is selected from S or P, X₂₉ is selected from S or P, and X₃₇ is selected from P or trans-Hyp; and the linker is: 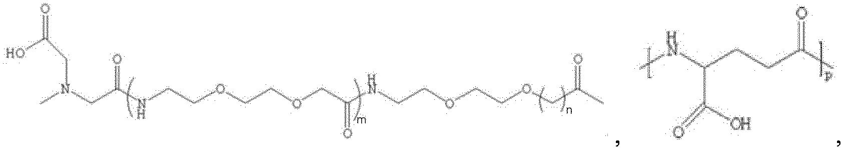 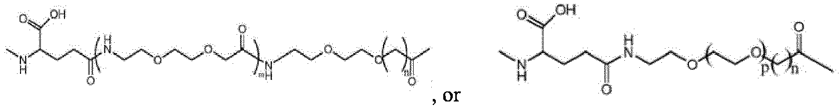
wherein m is 1 or 2; n is 1 or 2; p is any integer from 1 to 5; and the extension part is 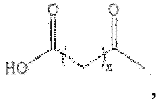 wherein x is any integer from 4 to 38.

In some embodiments, the polypeptide analog derivative of the present disclosure is selected from the following compounds as shown in Table 2, wherein PD065 is a Cagrilintide.

**Table 2.**

| Name of compound | Sequence of polypeptide | Modification site | Fatty acid-containing side chain |
|---|---|---|---|
| PD001 | RCNTATCATQRLAEFLRHSSKNLGPILPPTNV GSNTP-NH2 | 21K | 2*AEEA+γGlu+C20 diacid |
| PD002 | RCNTATCATQRLAEFLRHSSNKLGPILPPTNV GSNTP-NH2 | 22K | 2*AEEA+γGlu+C20 diacid |
| PD003 | RCNTATCATQRLAEFLRHSSNNKGPILPPTN VGSNTP-NH₂ | 23K | 2*AEEA+γGlu+C20 diacid |
| PD004 | RCNTATCATQRLAEFLRHSSNNLKPILPPTNV GSNTP-NH₂ | 24K | 2*AEEA+γGlu+C20 diacid |
| PD005 | RCNTATCATQRLAEFLRHSSNNLGKILPPTN VGSNTP-NH₂ | 25K | 2*AEEA+γGlu+C20 diacid |
| PD006 | RCNTATCATQRLAEFLRHSSKNLGPILPPTNV GSNTP-NH₂ | 21K | γGlu+C20 diacid |
| PD007 | RCNTATCATQRLAEFLRHSSNKLGPILPPTNV GSNTP-NH2 | 22K | γGlu+C20 diacid |
| PD008 | RCNTATCATQRLAEFLRHSSNNKGPILPPTN VGSNTP-NH₂ | 23K | γGlu+C20 diacid |
| PD009 | RCNTATCATQRLAEFLRHSSNNLKPILPPTNV GSNTP-NH2 | 24K | γGlu+C20 diacid |
| PD010 | RCNTATCATQRLAEFLRHSSNNLGKILPPTN VGSNTP-NH₂ | 25K | γGlu+C20 diacid |
| PD011 | RCNTATCATQRLAEFLRHSSNNLKPILSSTNV GSNTP-NH₂ | 24K | γGlu+C20 diacid |
| PD012 | RCNTATCATQRLAEFLRHSSNNLKAILPSTN VGSNTP-NH2 | 24K | γGlu+C20 diacid |
| PD013 | RCNTATCATQRLAEFLRHSSNNLKAILSPTN VGSNTP-NH₂ | 24K | γGlu+C20 diacid |
| PD014 | RCNTATCATQRLAEFLRHSSNNLKPILPSTNV GSNTP-NH₂ | 24K | γGlu+C20 diacid |
| PD015 | RCNTATCATQRLAEFLRHSSNNLKPILSPTNV GSNTP-NH₂ | 24K | γGlu+C20 diacid |
| PD016 | RCNTATCATQRLAEFLRHSSNNLKAILPPTN VGSNTP-NH₂ | 24K | γGlu+C20 diacid |
| PD017 | RCNTATCATQRLAEFLRHSSNNLKPILPPTNV GSNTY-NH2 | 24K | γGlu+C20 diacid |
| PD018 | RCNTATCATQRLAEFLRHSSNNLKAILSSTN VGSNTY-NH₂ | 24K | γGlu+C20 diacid |
| PD019 | RCNTATCATQRLAEFLRHSSNNLKPILSSTNV GSNTY-NH2 | 24K | γGlu+C20 diacid |
| PD020 | RCNTATCATQRLAEFLRHSSNNLKAILPSTN VGSNTY-NH₂ | 24K | γGlu+C20 diacid |
| PD021 | RCNTATCATQRLAEFLRHSSNNLKAILSPTN VGSNTY-NH2 | 24K | γGlu+C20 diacid |
| PD022 | RCNTATCATQRLAEFLRHSSNNLKPILPSTNV GSNTY-NH₂ | 24K | γGlu+C20 diacid |
| PD023 | RCNTATCATQRLAEFLRHSSNNLKPILSPTNV GSNTY-NH2 | 24K | γGlu+C20 diacid |
| PD024 | RCNTATCATQRLAEFLRHSSNNLKAILPPTN VGSNTY-NH₂ | 24K | γGlu+C20 diacid |
| PD025 | RCNTATCATQRLAEFLRHSSNNLKPILPPTNV GSGTP-NH2 | 24K | γGlu+C20 diacid |
| PD026 | RCNTATCATQRLAEFLRHSSNNLKAILSSTN VGSGTP-NH₂ | 24K | γGlu+C20 diacid |
| PD027 | RCNTATCATQRLAEFLRHSSNNLKPILSSTNV GSGTP-NH₂ | 24K | γGlu+C20 diacid |
| PD028 | RCNTATCATQRLAEFLRHSSNNLKAILPSTN VGSGTP-NH2 | 24K | γGlu+C20 diacid |
| PD029 | RCNTATCATQRLAEFLRHSSNNLKAILSPTN VGSGTP-NH₂ | 24K | γGlu+C20 diacid |
| PD030 | RCNTATCATQRLAEFLRHSSNNLKPILPSTNV GSGTP-NH2 | 24K | γGlu+C20 diacid |
| PD031 | RCNTATCATQRLAEFLRHSSNNLKPILSPTNV GSGTP-NH2 | 24K | γGlu+C20 diacid |
| PD032 | RCNTATCATQRLAEFLRHSSNNLKAILPPTN VGSGTP-NH2 | 24K | γGlu+C20 diacid |
| PD033 | KCNTATCATQRLAEFLRHSSNNLKPILPPTN VGSNTP-NH₂ | 24K | γGlu+C20 diacid |
| PD034 | KCNTATCATQRLAEFLRHSSNNLKPILSSTN VGSNTP-NH₂ | 24K | γGlu+C20 diacid |
| PD035 | KCNTATCATQRLAEFLRHSSNNLKAILPSTN VGSNTP-NH₂ | 24K | γGlu+C20 diacid |
| PD036 | KCNTATCATQRLAEFLRHSSNNLKAILSPTN VGSNTP-NH2 | 24K | γGlu+C20 diacid |
| PD037 | KCNTATCATQRLAEFLRHSSNNLKPILPSTN VGSNTP-NH₂ | 24K | γGlu+C20 diacid |
| PD038 | KCNTATCATQRLAEFLRHSSNNLKPILSPTN VGSNTP-NH2 | 24K | γGlu+C20 diacid |
| PD039 | KCNTATCATQRLAEFLRHSSNNLKAILPPTN VGSNTP-NH₂ | 24K | γGlu+C20 diacid |
| PD040 | RCNTATCATQRLAEFLRHSSNNLKPILPPTNV GSNT-trans-Hyp-NH₂ | 24K | γGlu+C20 diacid |
| PD041 | RCNTATCATQRLAEFLRHSSNNLKPILPPTNV GSGT-trans-Hyp-NH2 | 24K | γGlu+C20 diacid |
| PD042 | RCNTATCATQRLADFLRHSSNNLKPILPPTN VGSNTP-NH2 | 24K | γGlu+C20 diacid |
| PD043 | RCNTATCATQRLA-Aad-FLRHSSNNLKPILPP TNVGSNTP-NH2 | 24K | γGlu+C20 diacid |
| PD044 | RCNTATCATQRLA-Dab-FLRHSSNNLKPILPP TNVGSNTP-NH2 | 24K | γGlu+C20 diacid |
| PD045 | RCNTATCATQRLA-Dap-FLRHSSNNLKPILPP TNVGSNTP-NH2 | 24K | γGlu+C20 diacid |
| PD046 | RCNTATCATQRLAEFL-Orn-HSSNNLKPILPP TNVGSNTP-NH2 | 24K | γGlu+C20 diacid |
| PD047 | RCNTATCATQRLAEFLRHSSNNLKPILPPTNV GSNTP-NH₂ | 24K | γGlu+C20 diacid |
| PD048 | RCNTATCATQRLADFLRHSSNNLKPILPPTN VGSNTP-NH2 | 24K | γGlu+C20 diacid |
| PD049 | CNTATCATQRLAEFLRHSSNNLKPILPPTNV GSNTP-NH₂ | 23K | γGlu+C20 diacid |
| PD050 | CNTATCATQRLADFLRHSSNNLKPILPPTNV GSNTP-NH₂ | 23K | γGlu+C20 diacid |
| PD051 | RCNTATCATQRLAEFLRHSSNNLKPILPPTNV GSNT-trans-Hyp-NH₂ | 24K | γGlu+C20 diacid |
| PD052 | RCNTATCATQRLADFLRHSSNNLKPILPPTN VGSNT-trans-Hyp-NH₂ | 24K | γGlu+C20 diacid |
| PD053 | CNTATCATQRLAEFLRHSSNNLKPILPPTNV GSNT-trans-Hyp-NH₂ | 23K | γGlu+C20 diacid |
| PD054 | CNTATCATQRLADFLRHSSNNLKPILPPTNV GSNT-trans-Hyp-NH₂ | 23K | γGlu+C20 diacid |
| PD055 | RCNTATCATQRLAEFLRHSSNNLKPILPPTNV GSNT-cis-Hyp-NH2 | 24K | γGlu+C20 diacid |
| PD056 | RCNTATCATQRLADFLRHSSNNLKPILPPTN VGSNT-cis-Hyp-NH2 | 24K | γGlu+C20 diacid |
| PD057 | RCNTATCATQRLAEFLRHSSNNLKPILPPTNV GSNT-Pip-NH₂ | 24K | γGlu+C20 diacid |
| PD058 | RCNTATCATQRLADFLRHSSNNLKPILPPTN VGSNT-Pip-NH2 | 24K | γGlu+C20 diacid |
| PD059 | RCNTATCATQRLAEFLRHSSNNLKPILPPTNV GSNT-Aze-NH2 | 24K | γGlu+C20 diacid |
| PD060 | RCNTATCATQRLADFLRHSSNNLKPILPPTN VGSNT-Aze-NH₂ | 24K | γGlu+C20 diacid |
| PD061 | RCNTATCATQRLAEFLRHSSNNLKPILPPTNV GSNT-3-Am-Pro-NH₂ | 24K | γGlu+C20 diacid |
| PD062 | RCNTATCATQRLADFLRHSSNNLKPILPPTN VGSNT-3-Am-Pro-NH₂ | 24K | γGlu+C20 diacid |
| PD063 | RCNTATCATQRLAEFLRHSSNNLKPILPPTNV GSNT-αMePro-NH₂ | 24K | γGlu+C20 diacid |
| PD064 | RCNTATCATQRLADFLRHSSNNLKPILPPTN VGSNT-αMePro-NH₂ | 24K | γGlu+C20 diacid |
| PD065 | KCNTATCATQRLAEFLRHSSNNFGPILPPTNV GSNTP-NH₂ | N-terminal | γGlu+C20 diacid |
| PD066 | KCNTATCATQRLAEFLRHSSNNFGPILPPTNV GSNTP-NH₂ | 1K-ε amino | γGlu+C20 diacid |
| PD067 | KCNTATCATQRLADFLRHSSNNFGPILPPTN VGSNT-trans-Hyp-NH₂ | N-terminal | γGlu+C20 diacid |
| PD068 | KCNTATCATQRLADFLRHSSNNFGPILPPTN VGSNT-trans-Hyp-NH₂ | 1K-ε amino | γGlu+C20 diacid |
| PD069 | KCNTATCATQRLADFLRHSSNNLGPILPPTN VGSNT-trans-Hyp-NH₂ | N-terminal | γGlu+C20 diacid |
| PD070 | KCNTATCATQRLADFLRHSSNNLGPILPPTN VGSNT-trans-Hyp-NH₂ | 1K-ε amino | γGlu+C20 diacid |
| PD071 | KCNTATCATQRLADFLRHSSNNLKPILPPTN VGSNT-trans-Hyp-NH2 | N-terminal | γGlu+C20 diacid |
| PD072 | KCNTATCATQRLADFLRHSSNNLKPILPPTN VGSNT-trans-Hyp-NH₂ | 1K-ε amino | γGlu+C20 diacid |
| PD073 | Dap-CNTATCATQRLADFLRHSSNNLKPILPPT NVGSNT-trans-Hyp-NH2 | 24K | γGlu+C20 diacid |
| PD074 | Dab-CNTATCATQRLADFLRHSSNNLKPILPPT NVGSNT-trans-Hyp-NH2 | 24K | γGlu+C20 diacid |
| PD075 | Orn-CNTATCATQRLADFLRHSSNNLKPILPPT NVGSNT-trans-Hyp-NH2 | 24K | γGlu+C20 diacid |
| PD076 | KCNTATCATQRLADFLRHSSNNLKPILPPTN VGSNT-trans-Hyp-NH₂ | 24K | γGlu+C20 diacid |
| PD077 | Har-CNTATCATQRLADFLRHSSNNLKPILPPT NVGSNT-trans-Hyp-NH2 | 24K | γGlu+C20 diacid |
| PD078 | HCNTATCATQRLADFLRHSSNNLKPILPPTN VGSNT-trans-Hyp-NH₂ | 24K | γGlu+C20 diacid |
| PD079 | DCNTATCATQRLADFLRHSSNNLKPILPPTN VGSNT-trans-Hyp-NH₂ | 24K | γGlu+C20 diacid |
| PD080 | ECNTATCATQRLADFLRHSSNNLKPILPPTN VGSNT-trans-Hyp-NH₂ | 24K | γGlu+C20 diacid |
| PD081 | RCNTATCATQRLADFLRHSSNNLKPILPPTN VGSNT-trans-Hyp-NH2 | 24K | 2*AEEA+γGlu+ C20 diacid |
| PD082 | RCNTATCATQRLADFLRHSSNNKGPILPPTN VGSNT-trans-Hyp-NH₂ | 23K | γGlu+C20 diacid |
| PD083 | RCNTATCATQRLADFLRHSSNNKGPILPPTN VGSNT-trans-Hyp-NH2 | 23K | 2*AEEA+γGlu+ C20 diacid |
| PD084 | RCNTATCATQRLADFLRHSSNNKGPILPPTN VGSNT-trans-Hyp-NH2 | 23K | γGlu+C18 diacid |
| PD085 | RCNTATCATQRLADFLRHSSNNKGPILPPTN VGSNT-trans-Hyp-NH₂ | 23K | 2*AEEA+γGlu+ C18 diacid |
| PD086 | RCNTATCATQRLADFLRHSSNKLGPILPPTN VGSNT-trans-Hyp-NH2 | 22K | γGlu+C20 diacid |
| PD087 | RCNTATCATQRLADFLRHSSNKLGPILPPTN VGSNT-trans-Hyp-NH₂ | 22K | 2*AEEA+γGlu+ C20 diacid |
| PD088 | RCNTATCATQRLADFLRHSSNKLGPILPPTN VGSNT-trans-Hyp-NH₂ | 22K | γGlu+C18 diacid |
| PD089 | RCNTATCATQRLADFLRHSSNKLGPILPPTN VGSNT-trans-Hyp-NH₂ | 22K | 2*AEEA+γGlu+ C18 diacid |
| PD090 | RCNTATCATQRLADFLRHSSKNLGPILPPTN VGSNT-trans-Hyp-NH₂ | 21K | γGlu+C20 diacid |
| PD091 | RCNTATCATQRLADFLRHSSKNLGPILPPTN VGSNT-trans-Hyp-NH₂ | 21K | 2*AEEA+γGlu+ C20 diacid |
| PD092 | RCNTATCATQRLADFLRHSSKNLGPILPPTN VGSNT-trans-Hyp-NH2 | 21K | γGlu+C18 diacid |
| PD093 | RCNTATCATQRLADFLRHSSKNLGPILPPTN VGSNT-trans-Hyp-NH₂ | 21K | 2*AEEA+γGlu+ C18 diacid |
| PD094 | RCNTATCATQRLADFLRHSKNNLGPILPPTN VGSNT-trans-Hyp-NH2 | 20K | γGlu+C20 diacid |
| PD095 | RCNTATCATQRLADFLRHSKNNLGPILPPTN VGSNT-trans-Hyp-NH₂ | 20K | 2*AEEA+γGlu+ C20 diacid |
| PD096 | RCNTATCATQRLADFLRHSKNNLGPILPPTN VGSNT-trans-Hyp-NH2 | 20K | γGlu+C18 diacid |
| PD097 | RCNTATCATQRLADFLRHSKNNLGPILPPTN VGSNT-trans-Hyp-NH₂ | 20K | 2*AEEA+γGlu+ C18 diacid |
| PD098 | RCNTATCATQRLADFLRHKSNNLGPILPPTN VGSNT-trans-Hyp-NH₂ | 19K | γGlu+C20 diacid |
| PD099 | RCNTATCATQRLADFLRHKSNNLGPILPPTN VGSNT-trans-Hyp-NH₂ | 19K | 2*AEEA+γGlu+ C20 diacid |
| PD100 | RCNTATCATQRLADFLRHKSNNLGPILPPTN VGSNT-trans-Hyp-NH2 | 19K | γGlu+C18 diacid |
| PD101 | RCNTATCATQRLADFLRHKSNNLGPILPPTN VGSNT-trans-Hyp-NH2 | 19K | 2*AEEA+γGlu+ C18 diacid |
| PD102 | RCNTATCATQRLADFLRHSSNNLGKILPPTN VGSNT-trans-Hyp-NH2 | 25K | γGlu+C20 diacid |
| PD103 | RCNTATCATQRLADFLRHSSNNLGKILPPTN VGSNT-trans-Hyp-NH₂ | 25K | 2*AEEA+γGlu+ C20 diacid |
| PD104 | RCNTATCATQRLADFLRHSSNNLGKILPPTN VGSNT-trans-Hyp-NH2 | 25K | γGlu+C18 diacid |
| PD105 | RCNTATCATQRLADFLRHSSNNLGKILPPTN VGSNT-trans-Hyp-NH₂ | 25K | 2AEEA+γGlu+ C18 diacid |
| PD106 | RCNTATCATQRLADFLRHSSNNLKPILPPTN VGSNT-trans-Hyp-NH2 | 24K | AEEA+γGlu+ C20 diacid |
| PD107 | RCNTATCATQRLADFLRHSSNNLKPILPPTN VGSNT-trans-Hyp-NH₂ | 24K | γGlu+C18 diacid |
| PD108 | RCNTATCATQRLADFLRHSSNNLKPILPPTN VGSNT-trans-Hyp-NH₂ | 24K | AEEA+γGlu+ C18 diacid |
| PD109 | RCNTATCATQRLADFLRHSSNNLKPILPPTN VGSNT-trans-Hyp-NH2 | 24K | 2*AEEA+γGlu+ C18 diacid |
| PD110 | RCNTATCATQRLADFLRHSSNNKGPILPPTN VGSNT-trans-Hyp-NH2 | 23K | AEEA+γGlu+ C20 diacid |
| PD111 | RCNTATCATQRLADFLRHSSNNKGPILPPTN VGSNT-trans-Hyp-NH2 | 23K | AEEA+γGlu+ C18 diacid |
| PD112 | RCNTATCATQRLADFLRHSSNKLGPILPPTN VGSNT-trans-Hyp-NH2 | 22K | AEEA+γGlu+ C20 diacid |
| PD113 | RCNTATCATQRLADFLRHSSNKLGPILPPTN VGSNT-trans-Hyp-NH2 | 22K | AEEA+γGlu+ C18 diacid |
| PD114 | RCNTATCATQRLADFLRHSSKNLGPILPPTN VGSNT-trans-Hyp-NH2 | 21K | AEEA+γGlu+ C20 diacid |
| PD115 | RCNTATCATQRLADFLRHSSKNLGPILPPTN VGSNT-trans-Hyp-NH2 | 21K | AEEA+γGlu+ C18 diacid |
| PD116 | RCNTATCATQRLADFLRHSKNNLGPILPPTN VGSNT-trans-Hyp-NH2 | 20K | AEEA+γGlu+ C20 diacid |
| PD117 | RCNTATCATQRLADFLRHSKNNLGPILPPTN VGSNT-trans-Hyp-NH2 | 20K | AEEA+γGlu+ C18 diacid |
| PD118 | RCNTATCATQRLADFLRHKSNNLGPILPPTN VGSNT-trans-Hyp-NH₂ | 19K | AEEA+γGlu+ C20 diacid |
| PD119 | RCNTATCATQRLADFLRHKSNNLGPILPPTN VGSNT-trans-Hyp-NH₂ | 19K | AEEA+γGlu+ C18 diacid |
| PD120 | RCNTATCATQRLADFLRHSSNNLGKILPPTN VGSNT-trans-Hyp-NH2 | 25K | AEEA+γGlu+ C20 diacid |
| PD121 | RCNTATCATQRLADFLRHSSNNLGKILPPTN VGSNT-trans-Hyp-NH2 | 25K | AEEA+γGlu+ C18 diacid |
| PD122 | KCNTATCATQRLADFLRHSSNNLGPILPPTN VGSNT-trans-Hyp-NH₂ | N-terminal | AEEA+γGlu+ C20 diacid |
| PD123 | KCNTATCATQRLADFLRHSSNNLGPILPPTN VGSNT-trans-Hyp-NH₂ | N-terminal | 2*AEEA+γGlu+ C20 diacid |
| PD124 | KCNTATCATQRLADFLRHSSNNLGPILPPTN VGSNT-trans-Hyp-NH2 | N-terminal | γGlu+C18 **diacid** |
| PD125 | KCNTATCATQRLADFLRHSSNNLGPILPPTN VGSNT-trans-Hyp-NH₂ | N-terminal | AEEA+γGlu+C 18 diacid |
| PD126 | KCNTATCATQRLADFLRHSSNNLGPILPPTN VGSNT-trans-Hyp-NH₂ | N-terminal | 2*AEEA+γGlu+C18 diacid |
| PD127 | RCNTATCATQRLADFLRHSSNNLGPILPPTN VGSNT-trans-Hyp-NH2 | N-terminal | γGlu+C20 diacid |
| PD128 | HCNTATCATQRLADFLRHSSNNLGPILPPTN VGSNT-trans-Hyp-NH2 | N-terminal | γGlu+C20 diacid |
| PD129 | KCNTATCATQRLADFLRHSSNNLGPILPPTN VGSNT-trans-Hyp-NH₂ | N-terminal | C20 diacid |
| PD130 | KCNTATCATQRLADFLRHSSNNLGPILPPTN VGSNT-trans-Hyp-NH₂ | N-terminal | αGlu+C20 diacid |
| PD131 | KCNTATCATQRLADFLRHSSNNLGPILPPTN VGSNT-trans-Hyp-NH₂ | N-terminal | αAsp+C20 diacid |
| PD132 | KCNTATCATQRLADFLRHSSNNLGPILPPTN VGSNT-trans-Hyp-NH₂ | N-terminal | βAsp+C20 diacid |
| PD133 | KCNTATCATQRLADFLRHSSNNLGPILPPTN VGSNT-trans-Hyp-NH₂ | N-terminal | Trx+C20 diacid |
| PD134 | KCNTATCATQRLADFLRHSSNNLGPILPPTN VGSNT-trans-Hyp-NH₂ | N-terminal | Inp+C20 diacid |
| PD135 | KCNTATCATQRLADFLRHSSNNLGPILPPTN VGSNT-trans-Hyp-NH₂ | N-terminal, 1K-ε | γGlu+C10 diacid |
| PD136 | KCNTATCATQRLADFLRHSSNNLGPILPPTN VGSNT-trans-Hyp-NH₂ | N-terminal, 1K-ε | γGlu+C12 diacid |
| PD137 | KCNTATCATQRLADFLRHSSNNLGPILPPTN VGSNT-trans-Hyp-NH₂ | N-terminal, 1K-ε | γGlu+C14 diacid |
| PD138 | KCNTATCATQRLADFLRHSSNNLGPILPPTN VGSNT-trans-Hyp-NH2 | N-terminal, 1K-ε | γGlu+C16 diacid |
| PD139 | KCNTATCATQRLADFLRHSSNNLGPILPPTN VGSNT-trans-Hyp-NH₂ | N-terminal, 1K-ε | γGlu+C18 diacid |
| PD140 | RCNTATCATQRLADFLRHSSNNLKPILPPTN VGSNT-trans-Hyp-NH2 | N-terminal | γGlu+C20 diacid |
| PD141 | HCNTATCATQRLADFLRHSSNNLKPILPPTN VGSNT-trans-Hyp-NH₂ | N-terminal | γGlu+C20 diacid |
| PD142 | KCNTATCATQRLADFLRHSSNNLKPILPPTN VGSNT-trans-Hyp-NH₂ | N-terminal | C20 diacid |
| PD143 | KCNTATCATQRLADFLRHSSNNLKPILPPTN VGSNT-trans-Hyp-NH₂ | N-terminal | αGlu+C20 diacid |
| PD144 | KCNTATCATQRLADFLRHSSNNLKPILPPTN VGSNT-trans-Hyp-NH₂ | N-terminal | αAsp+C20 diacid |
| PD145 | KCNTATCATQRLADFLRHSSNNLKPILPPTN VGSNT-trans-Hyp-NH₂ | N-terminal | βAsp+C20 diacid |
| PD146 | KCNTATCATQRLADFLRHSSNNLKPILPPTN VGSNT-trans-Hyp-NH₂ | N-terminal | Trx+C20 diacid |
| PD147 | KCNTATCATQRLADFLRHSSNNLKPILPPTN VGSNT-trans-Hyp-NH₂ | N-terminal | Inp+C20 diacid |
| PD148 | KCNTATCATQRLADFLRHSSNNLKPILPPTN VGSNT-trans-Hyp-NH₂ | N-terminal, 1K-ε | γGlu+C10 diacid |
| PD149 | KCNTATCATQRLADFLRHSSNNLKPILPPTN VGSNT-trans-Hyp-NH2 | N-terminal, 1K-ε | γGlu+C12 diacid |
| PD150 | KCNTATCATQRLADFLRHSSNNLKPILPPTN VGSNT-trans-Hyp-NH₂ | N-terminal, 1K-ε | γGlu+C14 diacid |
| PD151 | KCNTATCATQRLADFLRHSSNNLKPILPPTN VGSNT-trans-Hyp-NH2 | N-terminal, 1K-ε | γGlu+C16 diacid |
| PD152 | KCNTATCATQRLADFLRHSSNNLKPILPPTN VGSNT-trans-Hyp-NH₂ | N-terminal, 1K-ε | γGlu+C18 diacid |
| PD153 | KCNTATCATQRLANFLVHSSNNFGPILPPTN VGSNTY-NH2 | N-terminal | γGlu+C20 diacid |
| PD154 | KCNTATCATQ RLADFLRHSS NNLKPILPPT NVGSNT-Aze-NH2 | N-terminal | γGlu+C20 diacid |
| PD155 | KCNTATCATQ RLADFLRHSS NNLGPILPPT NVGSNT-Aze-NH2 | N-terminal | γGlu+C20 diacid |
| PD156 | KCNTATCATQ RLANFLVHSS NNFGPILPPT NVGSNTY-NH2 | N-terminal | γGlu+C20 diacid |
| PD157 | KCNTATCATQ RLADFLRHSS NNLKPILPPT NVGSNT-Aze-NH2 | N-terminal | γGlu+C20 diacid |
| PD158 | KCNTATCATQ RLADFLRHSS NNLRPILPPT NVGSNT-trans-Hyp-NH₂ | N-terminal | γGlu+C20 diacid |
| PD159 | KCNTATCATQ RLADFLRHSS NNLHPILPPT NVGSNT-trans-Hyp-NH2 | N-terminal | γGlu+C20 diacid |
| PD160 | KCNTATCATQ RLADFLRHSS NNLKPILPPT NVGSNTP-NH2 | N-terminal | γGlu+C20 diacid |
| PD161 | KCNTATCATQ RLADFLRHSS NNLRPILPPT NVGSNTP-NH2 | N-terminal | γGlu+C20 diacid |
| PD162 | KCNTATCATQ RLADFLRHSS NNLHPILPPT NVGSNTP-NH2 | N-terminal | γGlu+C20 diacid |
| PD163 | KCNTATCATQ RLADFLRHSS NNLPPILPPT NVGSNTP-NH2 | N-terminal | γGlu+C20 diacid |
| PD164 | KCNTATCATQ RLADFLRHSS NNLDPILPPT NVGSNTP-NH2 | N-terminal | γGlu+C20 diacid |
| PD165 | KCNTATCATQ RLADFLRHSS NNLNPILPPT NVGSNTP-NH2 | N-terminal | γGlu+C20 diacid |
| PD166 | KCNTATCATQ RLADFLRHSS NNLSPILPPT NVGSNTP-NH2 | N-terminal | γGlu+C20 diacid |
| PD167 | KCNTATCATQ RLADFLRHSS NNL-αK-PILPPT NVGSNTP-NH2 | N-terminal | γGlu+C20 diacid |
| PD168 | KCNTATCATQ RLADFLRHSS NNL-Dap-PILPPT NVGSNTP-NH2 | N-terminal | γGlu+C20 diacid |
| PD169 | KCNTATCATQ RLADFLRHSS NNL-Dab-PILPPT NVGSNTP-NH2 | N-terminal | γGlu+C20 diacid |
| PD170 | KCNTATCATQ RLADFLRHSS NNL-Orn-PILPPT NVGSNTP-NH2 | N-terminal | γGlu+C20 diacid |
| PD171 | KCNTATCATQ RLADFLRHSS NNL-Cit-PILPPT NVGSNTP-NH2 | N-terminal | γGlu+C20 diacid |
| PD172 | KCNTATCATQ RLADFLRHSS NNL-Sar-PILPPT NVGSNTP-NH2 | N-terminal | γGlu+C20 diacid |
| PD173 | KCNTATCATQ RLADFLRHSS NNL-Trx-PILPPT NVGSNTP-NH2 | N-terminal | γGlu+C20 diacid |
| PD174 | KCNTATCATQ RLADFLRHSS NNL-AOC-PILPPT NVGSNTP-NH2 | N-terminal | γGlu+C20 diacid |
| PD175 | KCNTATCATQ RLADFLRHSS NNLGPILPPT NVGSNT-Aze-NH2 | N-terminal | γGlu+C20 diacid |
| PD176 | KCNTATCATQ RLADFLRHSS NNLKPILPPT NVGSNT-trans-Hyp-NH2 | N-terminal | γGlu+2*AEEA+ C20 diacid |
| PD177 | KCNTATCATQ RLADFLRHSS NNLKPILPPT NVGSNT-trans-Hyp-NH2 | N-terminal | 2*AEEA+Trx+C20 diacid |
| PD178 | KCNTATCATQ RLADFLRHSS NNLKPILPPT NVGSNT-trans-Hyp-NH2 | N-terminal | Trx+2*AEEA+ C20 diacid |
| PD179 | KCNTATCATQ RLADFLRHSS NNLKPILPPT NVGSNT-trans-Hyp-NH2 | N-terminal | 2*AEEA+Inp+ C20 diacid |
| PD180 | KCNTATCATQ RLADFLRHSS NNLKPILPPT NVGSNT-trans-Hyp-NH2 | N-terminal | Inp+2*AEEA+ C20 diacid |
| PD181 | KCNTATCATQ RLADFLRHSS NNLKPILPPT NVGSNT-trans-Hyp-NH2 | N-terminal | 2*AEEA+αAsp+C20 diacid |
| PD182 | KCNTATCATQ RLADFLRHSS NNLKPILPPT NVGSNT-trans-Hyp-NH2 | N-terminal | αAsp+2*AEEA+ C20 diacid |
| PD183 | KCNTATCATQ RLADFLRHSS NNLKPILPPT NVGSNT-trans-Hyp-NH2 | N-terminal | 2*AEEA+βAsp+ C20 diacid |
| PD184 | KCNTATCATQ RLADFLRHSS NNLKPILPPT NVGSNT-trans-Hyp-NH₂ | N-terminal | βAsp+2*AEEA+ C20 diacid |
| PD185 | KCNTATCATQ RLADFLRHSS NNLKPILPPT NVGSNT-trans-Hyp-NH2 | N-terminal | 8*PEG+γGlu+C20 diacid |
| PD186 | KCNTATCATQ RLADFLRHSS NNLKPILPPT NVGSNT-trans-Hyp-NH2 | N-terminal | γGlu+8*PEG+ C20 diacid |
| PD187 | KCNTATCATQ RLADFLRHSS NNLKPILPPT NVGSNT-trans-Hyp-NH2 | N-terminal | 2*AEEA +C20 diacid |
| PD188 | KCNTATCATQ RLADFLRHSS NNLKPILPPT NVGSNTP-NH2 | N-terminal | γGlu+2*AEEA+ C20 diacid |
| PD189 | KCNTATCATQ RLADFLRHSS NNLKPILPPT NVGSNTP-NH2 | N-terminal | 2*AEEA+Trx+ C20 diacid |
| PD190 | KCNTATCATQ RLADFLRHSS NNLKPILPPT NVGSNTP-NH2 | N-terminal | Trx+2*AEEA+ C20 diacid |
| PD191 | KCNTATCATQ RLADFLRHSS NNLKPILPPT NVGSNTP-NH2 | N-terminal | 2*AEEA +Inp+C20 diacid |
| PD192 | KCNTATCATQ RLADFLRHSS NNLKPILPPT NVGSNTP-NH2 | N-terminal | Inp+2*AEEA+ C20 diacid |
| PD193 | KCNTATCATQ RLADFLRHSS NNLKPILPPT NVGSNTP-NH2 | N-terminal | 2*AEEA +αAsp+C20 diacid |
| PD194 | KCNTATCATQ RLADFLRHSS NNLKPILPPT NVGSNTP-NH₂ | N-terminal | αAsp+2*AEEA+ C20 diacid |
| PD195 | KCNTATCATQ RLADFLRHSS NNLKPILPPT NVGSNTP-NH2 | N-terminal | 2*AEEA +βAsp+C20 diacid |
| PD196 | KCNTATCATQ RLADFLRHSS NNLKPILPPT NVGSNTP-NH2 | N-terminal | 2*AEEA+βAsp+C20 diacid |
| PD197 | RCNTA TCATQ RLADF LRHSS NNLRP ILPPT NVGSN TP-NH₂ | N-terminal | γGlu +C20 diacid |
| PD198 | KGGGG RCNTA TCATQ RLADF LRHSS NNLRP ILPPT NVGSN TP-NH2 | N-terminal | γGlu+ C20 diacid |

The compounds PD001 to PD198 represent polypeptide derivatives obtained by modifying the amino acid sequences with the fatty acid-containing side chains, wherein the fatty acid-containing side chain refers to the functional group capable of being used for coupling which is introduced into the amino acid side chain for modifying the amino acid in the present disclosure, such as the derivatives obtained by modifying sequences of corresponding polypeptides with corresponding fatty acid-containing side chains in Table 2. For example, the PD001 is a derivative obtained by modifying an amino acid K in a 21^{st} position of the sequence RCNTATCATQRLAEFLRHSSKNLGPILPPTNVGSNTP-NH₂ with the functional group capable of being used for coupling, that is, the side chain (2*AEEA+γGlu+C20 diacid). In general, C16 to C22 fatty diacid parts are located outside the fatty acid-containing side chain used for modification relative to the amino acid sequence, and at a far end of the side chain linked to the amino acid sequence.

In some embodiments, the polypeptide analog derivative of the present disclosure is preferably selected from the following derivatives: PD069, PD070, PD071, PD072, PD140, PD158, PD160, PD161, PD144, PD145, PD146, PD147 and PD187.

In some embodiments, the polypeptide analog derivative of the present disclosure is the compound PD069, and the PD069 has a structure below,

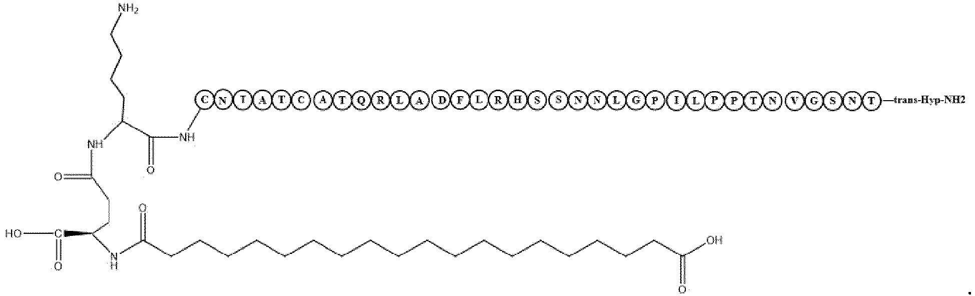

In some embodiments, the polypeptide analog derivative of the present disclosure is the compound PD070, and the PD070 has a structure below,

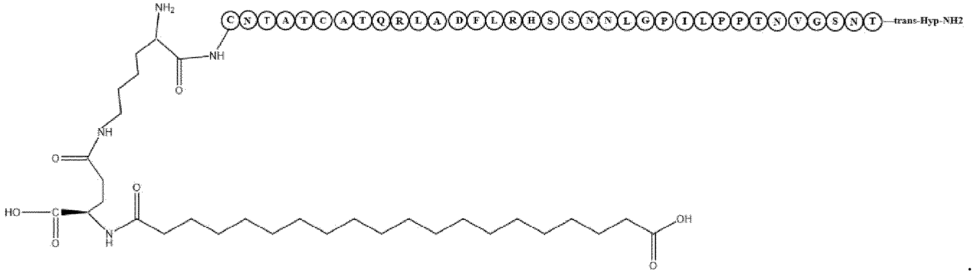

In some embodiments, the polypeptide analog derivative of the present disclosure is the compound PD071, and the PD071 has a structure below,

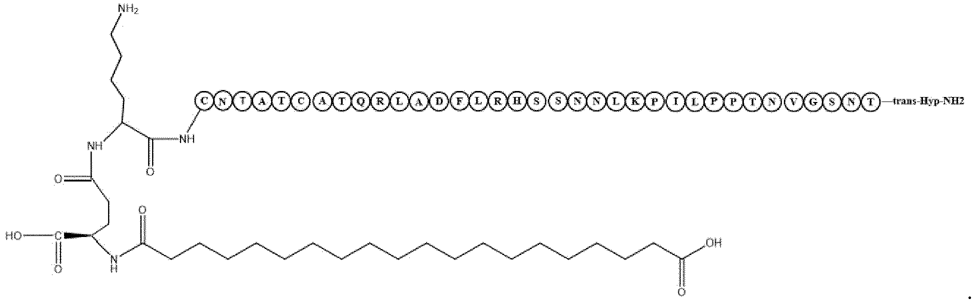

In some embodiments, the polypeptide analog derivative of the present disclosure is the compound PD072, and the PD072 has a structure below,

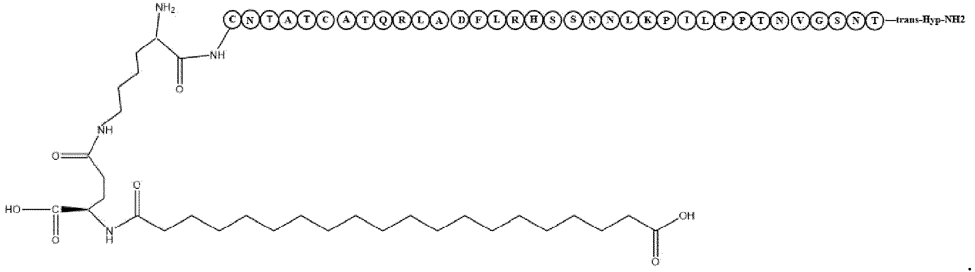

One aspect of the present disclosure further relates to a preparation method of the new amylin analog or the derivative thereof.

In some embodiments, the preparation of the new amylin analog or the derivative thereof may be implemented by conventional methods known to those skilled in the art. In general, a polypeptide molecule may be prepared by a chemical synthesis method or a biological fermentation method, or a combination of the two methods. For example, the polypeptide may be synthesized through a polypeptide gene sequence during synthesis, a corresponding polypeptide gene with a tag is inserted into a plasmid by a conventional method of genetic engineering to construct an expression plasmid, the expression plasmid is transferred into competent cells to construct expression cells, the expression cells containing a target polypeptide gene are screened, then cultured under suitable culture conditions, a culture product is collected, and a target polypeptide is finally obtained by a purification method. Further, an amino acid side chain of the target polypeptide may be modified by a chemical modification method, and the polypeptide analog derivative is obtained by purification.

In one aspect, the present disclosure further relates to a medicament or a pharmaceutical composition comprising the amylin analog or the derivative thereof above and a pharmaceutically acceptable salt of the above substance.

In some embodiments, the pharmaceutical composition of the present disclosure further comprises one or more oral delivery agents; preferably, the oral delivery agent is N-[8-(2-hydroxybenzoyl)amino]octanoic acid (NAC) salt; and further preferably, the oral delivery agent is PNAC, that is, potassium N-[8-(2-hydroxybenzoyl)amino] octanoate. The NAC salt or the PNAC salt of the oral delivery agent in the present disclosure should be understood as any crystal form capable of satisfying an oral delivery form of the composition of the present disclosure, such as the form in CN112661663B.

The PNAC salt has a structural formula (II) below:

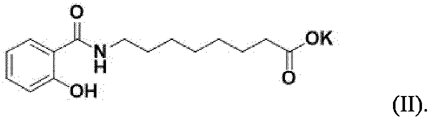

The NAC salt has a structural formula (I) below:

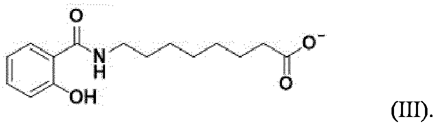

In some embodiments, the pharmaceutical composition of the present disclosure further comprises another or more pharmacologically active substances.

In some embodiments, the composition of the present disclosure further comprises a pharmaceutically acceptable excipient, preferably, the pharmaceutically acceptable excipient comprises, but is not limited to, one or more of a pharmaceutically acceptable carrier, a stabilizer, a dispersant, a cosolvent, a shaping agent, and the like. Generally, these substances are non-toxic, inert and pharmaceutically acceptable inactive substances.

In one aspect, the present disclosure provides use of the amylin analog or the derivative thereof, or the pharmaceutical composition above in preparation of a medicament for preventing and/or treating a disease, preferably, the use comprises a use in preparation of a medicament for preventing and/or treating an amylin receptor-related disease, and further preferably, the use comprises a use in preparation of a medicament for preventing and/or treating fat metabolism disorder, blood glucose metabolism disorder, a cardiovascular disease, a brain system disease, a mental system disease or a nervous system disease, comprising, but being not limited to, weight abnormality, hyperlipidemia, hypertension, arteriosclerosis, fatty liver, dyslipidemia, liver cirrhosis, a coronary heart disease, angina pectoris, myocardial infarction, an inflammatory bowel disease, dyspepsia and gastrointestinal ulcer, hyperglycemia, diabetes, impaired glucose tolerance, syndrome X, cognitive impairment, and stroke, preferably, the disease is the weight abnormality or the blood glucose metabolism disorder, and further preferably, the disease is the weight abnormality, comprising, but being not limited to, obesity or overweight.

In one aspect, the present disclosure provides a method for treating or/and preventing a disease, wherein the method comprises administering the amylin analog or the derivative thereof, the medicament or the pharmaceutical composition in the present disclosure to a subject in need, the disease is an amylin receptor-related disease, preferably, the disease is fat metabolism disorder, blood glucose metabolism disorder, a cardiovascular disease, a brain system disease, a mental system disease or a nervous system disease, comprising, but being not limited to, weight abnormality, hyperlipidemia, hypertension, arteriosclerosis, fatty liver, dyslipidemia, liver cirrhosis, a coronary heart disease, angina pectoris, myocardial infarction, an inflammatory bowel disease, dyspepsia and gastrointestinal ulcer, hyperglycemia, diabetes, impaired glucose tolerance, syndrome X, cognitive impairment, and stroke, further preferably, the disease is the weight abnormality or the blood glucose metabolism disorder, and more preferably, the disease is the weight abnormality, comprising, but being not limited to, obesity or overweight.

The amylin analog or the derivative thereof, the medicament or the pharmaceutical composition in the present disclosure may also be administered in combination with another or more pharmacologically active substances, the pharmacologically active substance comprises, but not is limited to, a body weight regulator, an anti-obesity agent, a lipid metabolism regulator, a blood glucose regulator, a hypertension therapeutic agent, a cardiovascular regulator, a brain system regulator, a mental system regulator or a nervous system regulator, preferably, the pharmacologically active substance is selected from: a GLP-1 receptor agonist (comprising, but being not limited to, GLP-1, a GLP-1 analog and a GLP-1 derivative), a GIP receptor agonist (comprising, but being not limited to, GIP, a GIP analog and a GIP derivative) and an insulin receptor agonist (comprising, but being not limited to, insulin, an insulin analog and an insulin derivative).

### Effects of invention

The polypeptide analog or the derivative thereof provided by the present disclosure has good agonistic activity on the insulin amyloid polypeptide receptor, has the characteristics of good stability, high activity, etc. at the same time, and can be used for treating and preventing diseases related to the insulin amyloid polypeptide receptor, such as body weight abnormality, especially obesity and overweight.

### DESCRIPTION OF THE DRAWINGS

The drawings are used for better understanding of the present disclosure, and do not constitute improper limitations to the present disclosure. In the drawings:
FIG. 1 shows stability experiment results of compounds PD065, PD069, PD070, PD071 and PD072 at 40°C on the 0 day, the 7^{th} day and the 15^{th} day respectively.
FIG. 2A shows change trends of weight loss effects of different derivatives in SD rats with time.
FIG. 2B shows change trends of influences of different derivatives on food intake in SD rats with time.
FIG. 2C shows weight change rates of SD rats in different derivative treatment groups in a group A at the 48^{th} hour.
FIG. 2D shows weight change rates of SD rats in different derivative treatment groups in a group B at the 48^{th} hour.
FIG. 3A shows change trends of weight loss effects of the compounds PD065 and PD069 at different concentrations in SD rats with time.
FIG. 3B shows change trends of weight loss effects of the compounds PD065 and PD070 at different concentrations in SD rats with time.
FIG. 3C shows change trends of weight loss effects of the compounds PD065 and PD071 at different concentrations in SD rats with time.
FIG. 3D shows change trends of weight loss effects of the compounds PD065 and PD072 at different concentrations in SD rats with time.
FIG. 4A shows change trends of influences of the compounds PD065 and PD071 at different concentrations on food intake in SD rats with time.
FIG. 4B shows change trends of influences of different derivative treatment groups at different concentrations on food intake in SD rats with time.
FIG. 5A shows change trends of weight loss effects of the compounds PD065 and PD187 in SD rats with time.
FIG. 5B shows change trends of weight loss effects of corresponding compounds in SD rats with time.
FIG. 5C shows change trends of influences of corresponding compounds on food intake in SD rats with time.
FIG. 6 shows pharmacokinetics of various polypeptides after Beagle dogs are orally administered with orally delivered compositions respectively containing different polypeptide derivatives.

### EMBODIMENT

Exemplary embodiments of the present disclosure are described hereinafter, and various details of the embodiments of the present application are included to help understanding, which should be considered as being exemplary only. Therefore, those of ordinary skills in the art should realize that various changes and modifications may be made to the embodiments described herein without departing from the scope and spirit of the present disclosure. In the same way, for the purpose of clarity and conciseness, the descriptions of well-known functions and structures are omitted in the following description.

### Abbreviation

Corresponding names or structural forms of some abbreviations used in the embodiments of the present disclosure are as shown in Table 3 below:

**Table 3.**

| Abbreviation | Structural formula | Abbreviation | Structural formula |
|---|---|---|---|
| *trans-Hyp* | 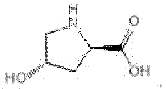 | Har | 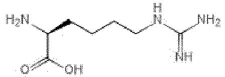 |
| *cis-Hyp* | 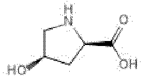 | Pip | 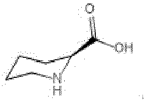 |
| 3-Am-Pro | 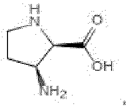 | Aze | 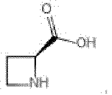 |
| Aad | 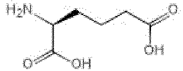 | αMePro | 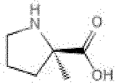 |
| Orn | 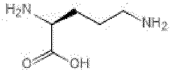 | Trx | 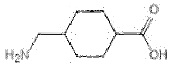 |
| Dab | 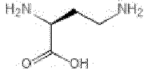 | Inp | 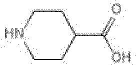 |
| Dap | 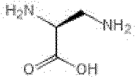 | AEEA | 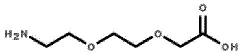 |

AA: Amino Acid; Boc: t-Butyloxy carbonyl;
DCM: dichloromethane; DMF: N,N-Dimethyl formamide; DIEA: N,N-Diisopropylethylamine; EDT: 1,2-Ethanedithiol; Fmoc: 9-fluorenylmethyloxycarbonyl; OtBu: tertiary butyl ester group; Pbf: 2,2,4,6,7-Pentamethyldihydrobenzofuran-5-sulfonyl chloride; Pip: Piperidine; TBTU: O-(Benzotriazol-l-yl)-N,N,N,N,-tetramethyluronium Tetrafluoroborate; tBu: tertiary butyl; TFA: Trifluoroacetic acid; TIS: Triisopropylsilane; Trt: Triphenylmethyl.

### Definition

The "Amylin" or "Amylin polypeptide" or "amylin" or "islet amyloid polypeptide" as used he -rein refers to a sequence of Lys-Cys-Asn-Thr-Ala-Thr-Cys-Ala-Thr-Gln-Arg-Leu-Ala-Asn-Phe-Leu-Val-His-Ser-Ser-Asn-Asn-Phe-Gly-Ala-Ile-Leu-Ser-Ser-Thr-Asn-Val-Gly-Ser-Asn-Thr-Tyr, or a variant/ analog, fragment or fusion protein of the above sequence which has basically maintaining the ago n-ism of an amylin receptor.

The "analog" as used herein refers to a compound in which at least one amino acid residue of a specific polypeptide molecule has been substituted by another amino acid residue, and/or at least one amino acid residue has been deleted, and/or at least one amino acid residue has been added to an N-terminal or a C-terminal, and/or at least one amino acid residue has been inserted between any two amino acids.

Unless otherwise clearly indicated in the context, the name of the specific polypeptide in the present disclosure, such as "Amylin", and the terms "polypeptide", "analog" and "variant" are interchangeable.

The "derivative" as used herein refers to a product obtained by modifying a functional group (such as an amino acid residue) of a biological macromolecule (such as a polypeptide and a protein) with certain compound or molecule. The modification comprises, but is not limited to, acylation, amidation, esterification and thioesterification.

The pharmaceutical composition according to the present disclosure may be administered in any suitable way, such as oral administration, nasal administration, intradermal administration, subcutaneous administration, intramuscular administration or intravenous administration.

The disease related to "fat metabolism disorder" in the present disclosure is a general name of related diseases caused by fat metabolism disorder, and the common disease is weight abnormality, such as obesity, and further comprises, for example, hyperlipidemia, hypertension, atherosclerosis, fatty liver, liver cirrhosis, a coronary heart disease, angina pectoris, myocardial infarction, an inflammatory bowel disease, dyspepsia, gastrointestinal ulcer, and the like.

The "weight abnormality" in the present disclosure means that, in the same evaluation system, a weight evaluation index of an object is beyond a normal average range of its population. In some embodiments, the weight abnormality refers to obesity. In some embodiments, the "weight loss" refers to reducing overweight or obesity.

### EXAMPLES

The present disclosure may be better understood with reference to the following examples. However, it should be understood that the following examples are only for illustrative purposes and should not be construed as limiting the scope of protection of the present disclosure in any way. Unless otherwise specified, the present disclosure may be implemented according to the methods listed in the experimental manuals such as "Molecular Cloning: A Laboratory Manual" and "Cell: A Laboratory Manual" as well as in the experimental guidelines of CFDA, which are familiar to those skilled in the art. The reagent raw materials used are all commercially available, and may be purchased through open channels.

### Example 1 Preparation of amylin derivative

A polypeptide was synthesized by a solid-phase organic synthesis method according to a Fmoc-protected amino acid strategy and an SPPS solid-phase synthesis technology, and was lysed, oxidized and purified to obtain a target product.

Taking a compound PD065 (Cagrilintide) as an example, a structural formula of the compound is as follows:

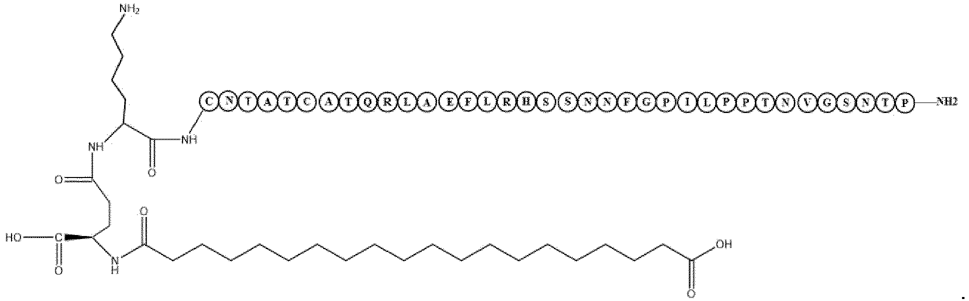

The synthesis process was as follows.

Solid-phase synthesis: amino acid connection was condensed from a C-terminal to an N-terminal (from right to left) by a method as shown in Table 4 with Fmoc-Linker MBHA Resin S=0.32 mmol/g through an Fmoc/tBu process according to the above sequence.

**Table 4. List of synthesis procedures**

| Procedure number | Solvent | Number of times | Time (min/time) |
|---|---|---|---|
| 1 | 20% Pip/DMF | 1 | 10 |
| 2 | 20% Pip/DMF | 1 | 5 |
| 3 | DMF | 5 | 1 |
| 4 | Sample and color development detection | | |
| 5 | AA/DIEA/TBTU | 1 | 60 |
| 6 | DMF | 4 | 1 |
| 7 | Sample and color development detection | | |
| Procedure description | 1. The procedures 1 to 7 were repeated until the synthesis of linear peptide was finished. | | |

An amino acid coupling order was as follows:
A-01Fmoc-Pro-OH, A-02Fmoc-Thr(tBu)-OH, A-03Fmoc-Asn(Trt)-OH, A-04Fmoc-Ser(tBu)-OH, A-05Fmoc-Gly-OH, A-06Fmoc-Val-OH, A-07Fmoc-Asn(Trt)-OH, A-08Fmoc-Thr(tBu)-OH, A-09Fmoc-Pro-OH, A-10Fmoc-Pro-OH, A-11Fmoc-Leu-OH, A-12Fmoc-Ile-OH, A-13Fmoc-Pro-OH, A-14Fmoc-Gly-OH, A-15Fmoc-Phe-OH, A-16Fmoc-Asn(Trt)-OH, A-17Fmoc-Asn(Trt)-OH, A-18Fmoc-Ser(tBu)-OH, A-19Fmoc-Ser(tBu)-OH, A-20Fmoc-His(Trt)-OH, A-21Fmoc-Arg(Pbf)-OH, A-22Fmoc-Leu-OH, A-23Fmoc-Phe-OH, A-24Fmoc-Glu(OtBu)-OH, A-25Fmoc-Ala-OH, A-26Fmoc-Leu-OH, A-27Fmoc-Arg(Pbf)-OH, A-28Fmoc-Gln(Trt)-OH, A-29Fmoc-Thr(tBu)-OH, A-30Fmoc-Ala-OH, A-31Fmoc-Cys(Trt)-OH, A-32Fmoc-Thr(tBu)-OH, A-33Fmoc-Ala-OH, A-34Fmoc-Thr(tBu)-OH, A-35Fmoc-Asn(Trt)-OH, A-36Fmoc-Cys(Trt)-OH, A-37Fmoc-Lys(Boc)-OH, A-38Fmoc-Glu-otbu, A-39C20 diacid.

Finally, the polypeptide derivative resin was formed, and the polypeptide derivative resin (peptide resin) was washed, transferred out, and dried to a constant weight to be lysed.

### Lysis of peptide resin:

Preparation of lysis reagents: amount of the lysis reagents was calculated according to 1 g peptide resin: 10 ml±2 ml, TFA: H₂O: EDT: TIS =95: 1: 2: 2. Required lysis reagents H₂O, TFA, EDT and TIS were sequentially added into a lysis reaction bottle, and a temperature of the lysis reagents was controlled at 0°C to 10°C; the lysis reagents were added into the peptide resin under stirring. After the temperature of the system was stabilized, the temperature of the lysis reagents was controlled at 25°C to 30°C, and the lysis reagents reacted under stirring for 2.5 hours. The lysis reagents were filtered out and precipitated with 5 times of liquid volume of glacial ethyl ether, and the precipitate was filtered out, washed with 3 times of liquid volume of glacial ethyl ether for 3 times, and dried at room temperature under a reduced pressure to obtain a solid crude product.

Oxidation of polypeptide: the crude product was finely ground, purified water was prepared, the finely ground crude product was slowly added under stirring, and an acetonitrile aqueous solution was dropwise added at the same time. After the crude product was completely added and dissolved, an iodine methanol solution was added and stirred for half an hour.

Purification and freeze-drying: the above oxidized liquid was filtered with a 0.45 µm microporous filtering membrane. The crude product was purified by a C-18 packing preparative column, and separated and purified at room temperature according to an appropriate gradient, and a target product was collected, analyzed and detected, and classified. The purity was required to be greater than or equal to 90%, an unqualified target product was collected, and separated and purified again according to an appropriate gradient, and then a qualified liquid peak was collected. The above qualified liquid sample was freeze-dried under reduced pressure to obtain refined polypeptide freeze-dried powder.

Compounds as shown in Table 2 were prepared by methods and steps similar to those of the compound PD065 (Cagrilintide).

### Example 2 In-vitro efficacy detection of amylin derivatives

The experiment was intended to detect the activity or efficacy of the amylin derivatives on a human amylin receptor in vitro by luciferase assay.

### 2.1 Construction of amylin receptor/CRE-luc cell line

CHO-K1/Ga15/AMY3 cells (a calcitonin receptor and a receptor-activated modified peptide RAMP had been constructed and purchased from GenScript) were transfected with a plasmid containing a luciferase expression cassette driven by multiple copies of cAMP response elements (CRE) by a standard method, and cultured in an F12 medium containing 200 µg/ml zeocin, 2 µg/mL puromycin, 100 µg/mL hygromycin and 400 µg/ml G418 to obtain the amylin receptor/CRE-luc cell line with stable transfection.

### 2.2 Luciferase assay of amylin

50 µL of growth medium (F12 medium containing 10% FBS) was added into each well of a white 96-well plate, and the stably transfected amylin/CRE-luc CHO cells were inoculated into the white 96-well plate at a density of about 20,000 cells/well for activity determination.

The freeze-dried powder obtained in Example 1 was dissolved in 20 mM phosphate buffer solution at pH 7.0, and diluted with the F12 medium containing 10% FBS to obtain a derivative sample at an initial concentration of 100 nM, the derivative sample was diluted with the F12 medium containing 10% FBS in gradient to obtain samples at 7 concentrations which were different by 10 times from 100 nM to 10⁻⁵ nM, 50 µL of sample determination solution at a corresponding concentration was added into each well, incubated at 37°C and 5% under CO₂ for 24 hours, then added with 100 µL of Luciferase per well, incubated for 3 minutes, and finally, a luminescence was determined on SpectraMax L (Molecular Devices) with SoftMax Pro 7.0.3 GxP software, and a standard curve was drawn according to a fluorescence value to calculate EC50. Results were shown in Table 5.

**Table 5.**

| Serial number of | EC50/nM | Serial number of | EC50/nM | Serial number of | EC50/nM | Serial number of | EC50/n M | Serial number of | EC50/nM |
|---|---|---|---|---|---|---|---|---|---|
| compound | | compound | | compound | | compound | | compound | |
| PD001 | 0.042 | PD063 | 0.101 | PD095 | 0.047 | PD156 | 0.029 | PD191 | 0.061 |
| PD002 | 0.119 | PD064 | 0.055 | PD096 | 0.042 | PD158 | 0.022 | PD192 | 0.087 |
| PD003 | 0.14 | PD065 | 0.038 | PD097 | 0.014 | PD159 | 0.037 | PD193 | 0.074 |
| PD004 | 0.102 | PD066 | 0.044 | PD098 | 0.077 | PD160 | 0.035 | PD194 | 0.074 |
| PD006 | 0.129 | PD067 | 0.034 | PD099 | 0.033 | PD161 | 0.026 | PD195 | 0.025 |
| PD007 | 0.205 | PD068 | 0.032 | PD100 | 0.06 | PD162 | 0.035 | PD196 | 0.064 |
| PD008 | 0.281 | PD069 | 0.039 | PD101 | 0.012 | PD163 | 0.084 | PD197 | 0.025 |
| PD009 | 0.067 | PD070 | 0.038 | PD102 | 0.033 | PD164 | 0.111 | PD198 | 0.029 |
| PD017 | 0.772 | PD071 | 0.028 | PD103 | 0.033 | PD165 | 0.05 | / | / |
| PD040 | 0.118 | PD072 | 0.027 | PD104 | 0.011 | PD166 | 0.054 | / | / |
| PD041 | 0.259 | PD073 | 0.063 | PD105 | 0.033 | PD167 | 0.05 | / | / |
| PD042 | 0.111 | PD074 | 0.066 | PD106 | 0.026 | PD168 | 0.041 | / | / |
| PD043 | 1.13 | PD075 | 0.048 | PD107 | 0.027 | PD169 | 0.039 | / | / |
| PD044 | 2.617 | PD076 | 0.04 | PD108 | 0.028 | PD170 | 0.041 | / | / |
| PD045 | 0.364 | PD077 | 0.034 | PD109 | 0.01 | PD171 | 0.053 | / | / |
| PD046 | 1.676 | PD078 | 0.035 | PD110 | 0.039 | PD172 | 0.075 | / | / |
| PD047 | 0.073 | PD079 | 0.131 | PD111 | 0.008 | PD173 | 0.064 | / | / |
| PD048 | 0.047 | PD080 | 0.11 | PD112 | 0.03 | PD174 | 0.046 | / | / |
| PD049 | 0.215 | PD081 | 0.031 | PD113 | 0.017 | PD176 | 0.029 | / | / |
| PD050 | 0.098 | PD082 | 0.038 | PD114 | 0.029 | PD177 | 0.024 | / | / |
| PD051 | 0.039 | PD083 | 0.037 | PD115 | 0.011 | PD178 | 0.017 | / | / |
| PD052 | 0.03 | PD084 | 0.008 | PD116 | 0.033 | PD179 | 0.017 | / | / |
| PD053 | 0.075 | PD085 | 0.005 | PD117 | 0.014 | PD180 | 0.016 | / | / |
| PD054 | 0.049 | PD086 | 0.033 | PD118 | 0.032 | PD181 | 0.022 | / | / |
| PD055 | 0.909 | PD087 | 0.032 | PD119 | 0.024 | PD182 | 0.034 | / | / |
| PD056 | 0.586 | PD088 | 0.013 | PD120 | 0.023 | PD183 | 0.04 | / | / |
| PD057 | 0.793 | PD089 | 0.018 | PD121 | 0.018 | PD184 | 0.037 | / | / |
| PD058 | 0.319 | PD090 | 0.031 | PD122 | 0.048 | PD186 | 0.052 | / | / |
| PD059 | 0.939 | PD091 | 0.03 | PD123 | 0.068 | PD187 | 0.016 | / | / |
| PD060 | 0.246 | PD092 | 0.016 | PD124 | 0.008 | PD188 | 0.025 | / | / |
| PD061 | 0.815 | PD093 | 0.019 | PD125 | 0.01 | PD189 | 0.029 | / | / |
| PD062 | 0.374 | PD094 | 0.034 | PD126 | 0.028 | PD190 | 0.017 | / | / |

Results in Table 5 showed that the polypeptide derivatives of the present disclosure all had good activity to the human amylin receptor.

### Example 3 Freeze-thaw stability of derivatives

The freeze-dried powder of the polypeptide analog derivative prepared in Example 1 was mixed with a buffer solution according to a mass-volume ratio of 0.2 mg/ml, the polypeptide was dissolved with 20 mM phosphate buffer solution (PB) (pH 7.0), the sample was stored at -20°C for overnight storage, and then the sample was taken out to be restored to room temperature for thawing. During the experiment, property changes of the polypeptide after dissolution and freezing and thawing were observed and recorded respectively, and concentrations of the polypeptide before and after the freezing and thawing were detected by the following liquid chromatography method:
Materials and equipment: high-performance liquid chromatographic instrument (Agilent 1200), ultrapure water (18.2 MΩ self-manufactured ultrapure water), acetonitrile (HPLC grade), and trifluoroacetic acid (HPLC grade); and reversed-phase chromatographic column: Sepax Bio-C4 4.6*100mm 5 µm 300Å.
Sample preparation: a certain amount of polypeptide was dissolved with 20 mM PB buffer solution at pH 7.4, and in the case of poor solubility, a small amount of 0.1 M NaOH could be added to adjust the pH to 8.0 for complete dissolution, and a concentration of the solution was controlled between 0.5 mg/ml and 2 mg/ml. A sample to be tested was centrifuged at 10,000 rpm and 4°C for 3 minutes, and a supernatant was taken and added into a liquid-phase sample injection bottle.
Chromatographic conditions: flow rate: 1.0 ml/min; automatic sample injection temperature: 15°C; column temperature: 15°C; detection wavelength: 280 nm; mobile phase A: 100% H₂O+0.05%TFA; and mobile phase b: 100% CAN. Elution gradients were as shown in the following table:

**Table 6 List of elution gradients of liquid chromatography**

| | | | | | |
|---|---|---|---|---|---|
| Time (min) | 0 | 10 | 14 | 15 | 25 |
| B% | 5 | 35 | 70 | 5 | 5 |

A freeze-thaw yield was calculated by the following formula: freeze-thaw yield = concentration of polypeptide after the freezing and thawing/concentration of polypeptide before the freezing and thawing. Related results were as shown in Table 7.

**Table 7.**

| Name of compound | Dissolution property | Freeze storage property | Freeze storage yield | Name of compound | Dissolution property | Freeze storage property | Freeze storage yield |
|---|---|---|---|---|---|---|---|
| PD001 | Clear | Clear | / | PD078 | Clear | Clear | 67.85% |
| PD002 | Clear | Clear | / | PD079 | Clear | Clear | 96.15% |
| PD003 | Clear | Clear | / | PD080 | Clear | Clear | 90.06% |
| PD004 | Clear | Clear | / | PD081 | Clear | Clear | 76.82% |
| PD005 | Clear | Clear | / | PD082 | / | / | / |
| PD006 | Clear | Clear | / | PD083 | Clear | Clear | 79.98% |
| PD007 | Clear | Clear | / | PD084 | / | / | / |
| PD008 | Clear | Clear | / | PD085 | Clear | Clear | 76.70% |
| PD009 | Clear | Clear | / | PD086 | Clear | Clear | 69.09% |
| PD010 | Clear | Clear | / | PD087 | Clear | Clear | 102.65% |
| PD011 | Clear | Slightly muddy | 15.33% | PD088 | Clear | Clear | 120.04% |
| PD012 | Muddy | Muddy | 0.00% | PD089 | Clear | Clear | 101.91% |
| PD013 | Clear | Muddy | 18.78% | PD090 | Clear | Clear | 99.24% |
| PD014 | Clear | Clear | 95.77% | PD091 | Clear | Clear | 101.14% |
| PD015 | Clear | Slightly muddy | 0.00% | PD092 | Clear | Clear | 101.74% |
| PD016 | Clear | Clear | 75.53% | PD093 | Clear | Clear | 99.86% |
| PD017 | Clear | Muddy | 100.81% | PD094 | Clear | Clear | 102.94% |
| PD018 | Muddy | Muddy | 33.24% | PD095 | Clear | Clear | 95.26% |
| PD019 | Muddy | Muddy | No peak | PD096 | Clear | Clear | 101.99% |
| PD020 | Slightly muddy | Muddy | 0.00% | PD097 | Clear | Clear | 98.20% |
| PD021 | Muddy | Muddy | 21.35% | PD098 | / | / | / |
| PD022 | Muddy | Muddy | 72.10% | PD099 | Clear | Clear | 102.80% |
| PD023 | Muddy | Muddy | 64.54% | PD100 | Clear | Clear | 102.97% |
| PD024 | Muddy | Muddy | 79.10% | PD101 | Clear | Clear | 97.67% |
| PD025 | Clear | Muddy | 102.64% | PD102 | Clear | Clear | 97.43% |
| PD026 | / | / | / | PD103 | Clear | Clear | 98.68% |
| PD027 | Muddy | Muddy | 58.88% | PD104 | Clear | Clear | 99.05% |
| PD028 | Muddy | Muddy | 0.00% | PD105 | Clear | Clear | 111.06% |
| PD029 | Muddy | Muddy | 27.17% | PD106 | Clear | Clear | 99.24% |
| PD030 | Clear | Clear | 94.86% | PD107 | Clear | Clear | 111.06% |
| PD031 | Muddy | Muddy | 81.43% | PD108 | Clear | Clear | 96.86% |
| PD032 | Clear | Clear | 103.57% | PD109 | Clear | Clear | 100.53% |
| PD033 | Clear | Clear | 69.52% | PD110 | Clear | Clear | 109.34% |
| PD034 | Clear | Muddy | 89.35% | PD111 | Clear | Clear | 105.17% |
| PD035 | Muddy | Muddy | 0.00% | PD112 | Clear | Clear | 98.62% |
| PD036 | Muddy | Muddy | 0.00% | PD113 | Clear | Clear | 77.90% |
| PD037 | Muddy | Muddy | 98.81% | PD114 | Clear | Clear | 101.02% |
| PD038 | Muddy | Muddy | 100.87% | PD115 | Clear | Clear | 104.27% |
| PD039 | Clear | Clear | 96.97% | PD116 | Clear | Clear | 102.48% |
| PD040 | Clear | Clear | 95.62% | PD117 | Clear | Clear | 93.49% |
| PD041 | Clear | Clear | 96.58% | PD118 | Clear | Clear | 104.70% |
| PD042 | Clear | Clear | 95.19% | PD119 | Clear | Clear | 103.04% |
| PD043 | Clear | Clear | 49.45% | PD120 | Clear | Clear | 103.41% |
| PD044 | Clear | Clear | 84.90% | PD121 | Clear | Clear | 102.87% |
| PD045 | Clear | Clear | 95.59% | PD122 | Clear | Clear | 103.66% |
| PD046 | Clear | Clear | 101.35% | PD123 | Clear | Clear | 102.11% |
| PD047 | Clear | Clear | 0.00% | PD124 | Clear | Clear | 91.04% |
| PD048 | Clear | Clear | 102.16% | PD125 | Clear | Clear | 95.59% |
| PD049 | Clear | Clear | 99.00% | PD126 | Clear | Clear | 99.24% |
| PD050 | Clear | Clear | 99.66% | PD127 | Clear | Clear | 101.18% |
| PD051 | Clear | Clear | 99.71% | PD128 | Clear | Clear | 97.05% |
| PD052 | Clear | Clear | 96.53% | PD129 | Clear | Clear | 106.67% |
| PD053 | Clear | Clear | 104.17% | PD130 | Clear | Clear | 107.64% |
| PD054 | Clear | Clear | 103.78% | PD131 | Clear | Clear | 108.14% |
| PD055 | Clear | Clear | 99.75% | PD132 | Clear | Clear | 101.01% |
| PD056 | Clear | Clear | 101.22% | PD133 | Clear | Clear | 98.24% |
| PD057 | Clear | Clear | 99.70% | PD134 | Clear | Clear | 101.39% |
| PD058 | Clear | Clear | 98.34% | PD135 | Clear | Clear | 110.57% |
| PD059 | Clear | Clear | 103.57% | PD136 | Clear | Clear | 108.07% |
| PD060 | Clear | Clear | 104.35% | PD137 | Clear | Clear | 102.20% |
| PD061 | Clear | Clear | 100.32% | PD138 | Clear | Clear | 106.03% |
| PD062 | Clear | Clear | 102.63% | PD139 | Clear | Clear | 104.78% |
| PD063 | Clear | Clear | 107.52% | PD140 | Clear | Clear | 100.18% |
| PD064 | Clear | Clear | 103.04% | PD141 | Clear | Clear | 96.57% |
| PD065 | Clear | Clear | 93.44% | PD142 | Clear | Clear | 101.09% |
| PD066 | Clear | Clear | 93.44% | PD143 | Clear | Clear | 118.11% |
| PD067 | Clear | Clear | 98.84% | PD144 | Clear | Clear | 101.98% |
| PD068 | Clear | Clear | 97.55% | PD145 | Clear | Clear | 100.88% |
| PD069 | Clear | Clear | 100.00% | PD146 | Clear | Clear | 101.09% |
| PD070 | Clear | Clear | 100.00% | PD147 | Clear | Clear | 106.44% |
| PD071 | Clear | Clear | 100.80% | PD148 | Clear | Clear | 103.34% |
| PD072 | Clear | Clear | 103.19% | PD149 | Clear | Clear | 99.99% |
| PD073 | Clear | Clear | 155.32% | PD150 | Clear | Clear | 103.74% |
| PD074 | Clear | Clear | 66.34% | PD151 | Clear | Clear | 101.88% |
| PD075 | Clear | Clear | 56.70% | PD152 | Clear | Clear | 97.96% |
| PD076 | Clear | Clear | 52.18% | PD153 | / | / | / |
| PD077 | Clear | Clear | 85.60% | PD154 | / | / | / |

As shown in Table 7, most of the polypeptide derivatives in the present disclosure had good freeze-thaw stability and yield.

### Example 4 Study on 40°C stability of derivatives

Compound PD065, PD069, PD070, PD071 or PD072 was mixed with sodium dihydrogen phosphate (1.42 mg/ml) according to 1.34 mg/ml, and dissolved in ultrapure water, pH of the solution was adjusted to about 7.4 with hydrochloric acid/sodium hydroxide, and then the solution was filtered into a sterilized penicillin bottle with a 0.22 µm sterile filter in an ultra-clean workbench. The penicillin bottle after covering was placed in a stability test box at 40°C.

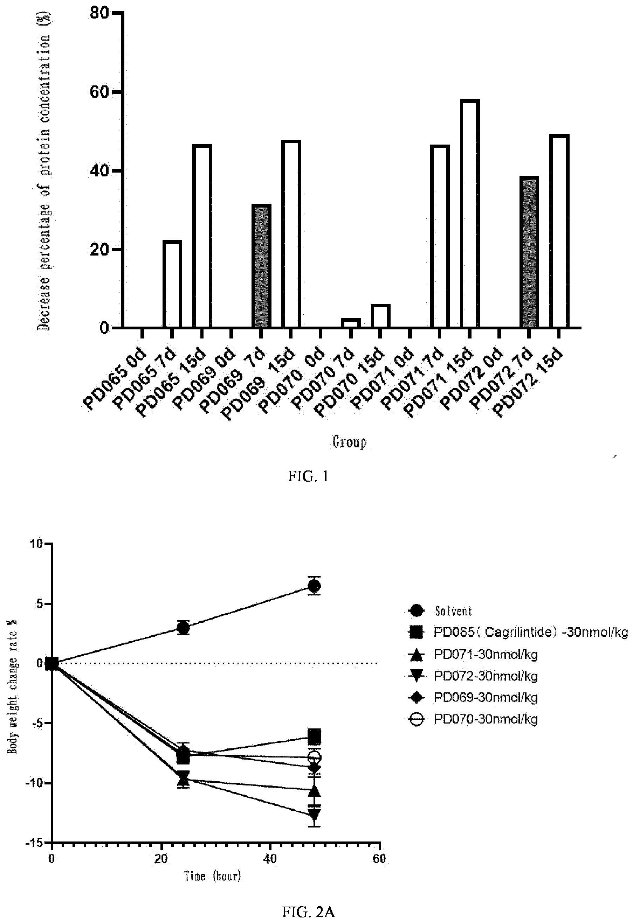

Before testing, a sample was centrifuged at 10,000 rpm and 4°C for 3 minutes, and a supernatant was taken and added into a liquid-phase sample injection bottle. The sample was detected by the liquid phase method in Example 3. Results were as shown in Table 8 and FIG. 1.

**Table 8.**

| Name of sample and number of days | Retention time (min) | Protein concentration (mg/ml) | Decrease percentage of protein concentration |
|---|---|---|---|
| PD065 (0d) | 12.95 | 0.81 | 0.00% |
| PD065 (7d) | 12.96 | 0.63 | 22.35% |
| PD065 (15d) | 12.972 | 0.43 | 46.78% |
| PD069 (0d) | 12.842 | 1.01 | 0.00% |
| PD069 (7d) | 12.853 | 0.69 | 31.57% |
| PD069 (15d) | 12.86 | 0.52 | 47.84% |
| PD070 (0d) | 13.007 | 0.98 | 0.00% |
| PD070 (7d) | 13.01 | 0.95 | 2.53% |
| PD070 (15d) | 13.01 | 0.92 | 6.28% |
| PD071 (0d) | 12.718 | 0.71 | 0.00% |
| PD071 (7d) | 12.745 | 0.38 | 46.69% |
| PD071 (15d) | 12.768 | 0.30 | 58.16% |
| PD072 (0d) | 12.867 | 0.96 | 0.00% |
| PD072 (7d) | 12.893 | 0.59 | 38.60% |
| PD072 (15d) | 12.883 | 0.49 | 49.27% |

As shown in Table 8, the thermal stability of the polypeptide derivatives in the present disclosure was basically equivalent to the thermal stability of the PD065, and surprisingly, the compound PD070 showed an extremely low decrease percentage of protein concentration.

### Example 5 Study on weight loss efficacy of derivatives

SD (Sprague Dawley) rats weighing 200 g to 250 g were selected for this experimental study. Before the experiment, the rats arrived for at least 10 days to 14 days to adapt to the experimental environment. During this period, the rats were fed for two weeks in a reversed light/dark stage (meaning that the light was turned off during the day and turned on during the night) after arrival, and were fed separately in the first week of arrival to ensure accurate data and high test sensitivity, and the rats were free to obtain food and water during the whole adaptation period and experimental period. There were an experimental group A: A1, A2, A3, A4, A5 and A6, and an experimental group B: B1, B2, B3, B4 and B5, with 5 to 8 rats in each treatment group of the derivative, and the rats in each group were subcutaneously administered with a derivative or a solvent by a dosage of 30 nmol/kg. The administration time of each group was recorded. After administration, the rats were put back into their cages in which the rats could get food and water. Food consumption and weight changes of the rats were recorded every 24 hours by online recording or manual recording.

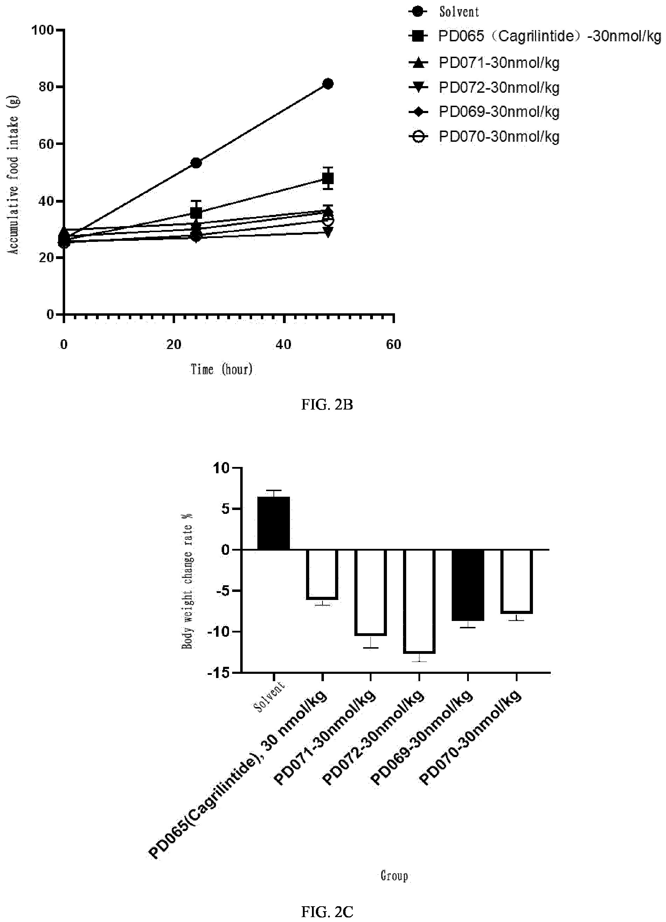

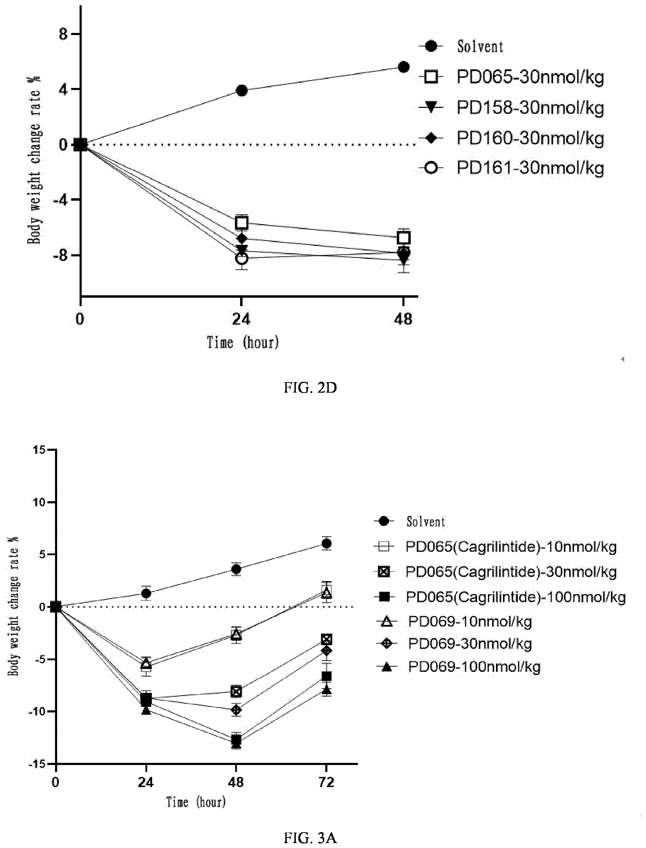

Weight and food intake changes of the SD rats in each group were as shown in FIG. 2A to FIG. 2D, Table 9 and Table 10.

**Table 9.**

| .Group | Treatment group | Weight change rate at 48^{th} hour |
|---|---|---|
| A1 | Solvent | 6.50 |
| A2 | PD065 (Cagrilintide) | -6.13 |
| A3 | PD069 | -8.70 |
| A4 | PD070 | -7.86 |
| A5 | PD071 | -10.58 |
| A6 | PD072 | -12.75 |

**Table 10.**

| Group | Treatment group | Weight change rate at 48^{th} hour |
|---|---|---|
| B1 | Solvent | 5.61 |
| B2 | PD065 (Cagrilintide) | -6.74 |
| B3 | PD158 | -8.37 |
| B4 | PD160 | -7.87 |
| B5 | PD161 | -7.81 |

Experimental results showed that, compared with a weight reduction effect of the PD065, the polypeptide derivatives in the present disclosure showed better weight reduction ability, which could even reach more than twice the weight reduction effect of the PD065.

### Example 6 Study on weight loss effect of dosage of derivatives

SD rats obtained by the same method as in Example 5 were used in this experimental study. There were an experimental group C: C1 to C13, and an experimental group D: D1 to D7, with 5 to 8 rats in each group of the derivative test, and according to administration schemes as shown in Table 11 and Table 12, the rats in each group were subcutaneously administered with a derivative or a solvent by a dosage of 10 nmol/kg, 20 nmol/kg or 30 nmol/kg. The administration time of each group was recorded. After administration, the rats were put back into their cages in which the rats could get food and water. Food consumption and weight changes of the rats were recorded every 24 hours by online recording or manual recording.

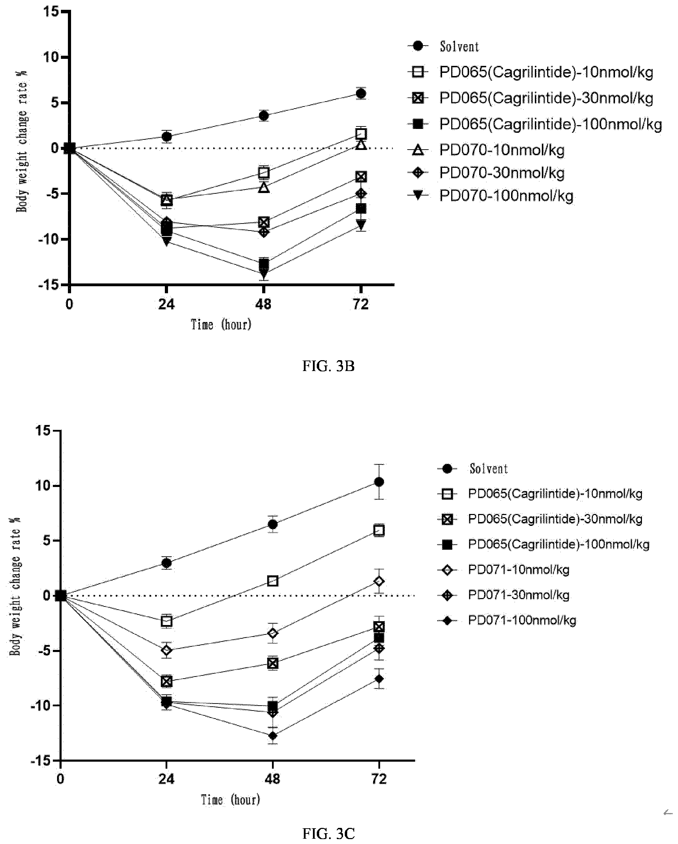

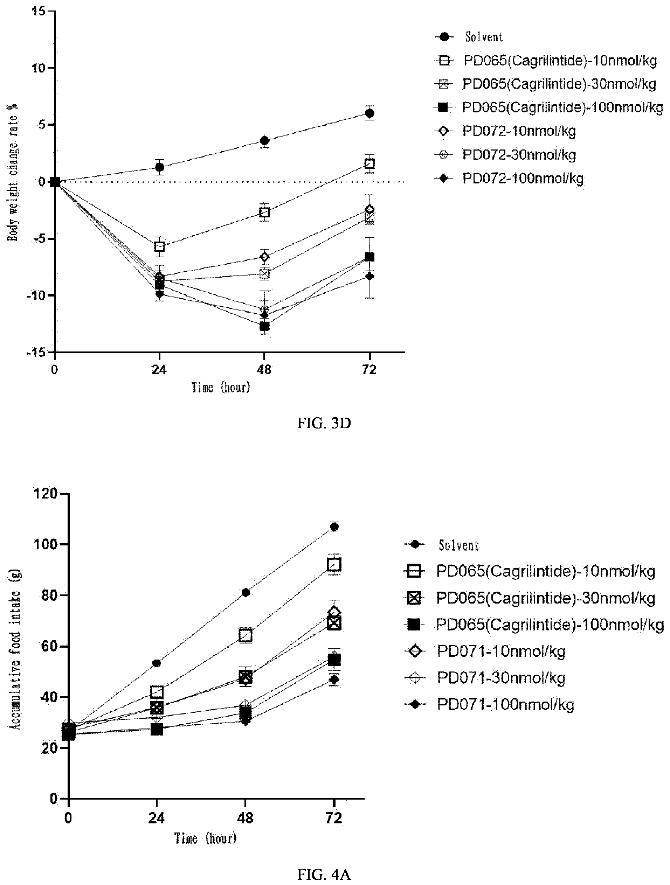

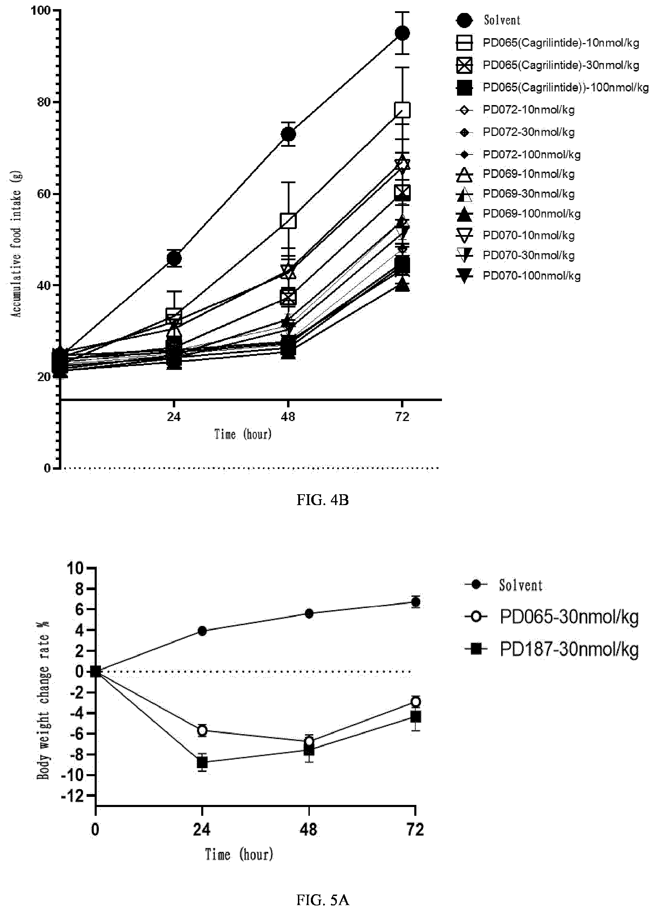

Weight and cumulative food intake changes of the SD rats in each group were as shown in FIG. 3A to FIG. 3D, FIG. 4A to FIG. 4B, Table 11 and Table 12.

**Table 11.**

| Group | Active ingredient and dosage | Weight change rate at 72^{nd} hour (%) | Cumulative food intake at 72^{nd} hour (g) |
|---|---|---|---|
| C1 | Solvent | 6.03 | 95.11 |
| C2 | PD065 (Cagrilintide), 10 nmol/kg | 1.59 | 78.31 |
| C3 | PD065 (Cagrilintide), 30 nmol/kg | 0.46 | 60.22 |
| C4 | PD065 (Cagrilintide), 100 nmol/kg | -6.61 | 44.41 |
| C5 | PD069, 10 nmol/kg | 1.36 | 67.05 |
| C6 | PD069, 30 nmol/kg | -4.16 | 54.14 |
| C7 | PD069, 100 nmol/kg | -7.88 | 40.5 |
| C8 | PD070, 10 nmol/kg | 0.47 | 65.71 |
| C9 | PD070, 30 nmol/kg | -4.96 | 51.4 |
| C10 | PD070, 100 nmol/kg | -8.47 | 45.04 |
| C11 | PD072, 10 nmol/kg | -2.43 | 53.99 |
| C12 | PD072, 30 nmol/kg | -6.61 | 47.96 |
| C13 | PD072, 100 nmol/kg | -8.30 | 44.84 |

**Table 12.**

| Group | Active ingredient and dosage | Weight change rate at 72^{nd} hour (%) | Cumulative food intake at 72^{nd} hour (g) |
|---|---|---|---|
| D1 | Solvent | 10.36 | 81.17 |
| D2 | PD065 (Cagrilintide), 10 nmol/kg | 5.95 | 64.27 |
| D3 | PD065 (Cagrilintide), 30 nmol/kg | 2.81 | 48.00 |
| D4 | PD065 (Cagrilintide), 100 nmol/kg | -3.81 | 33.89 |
| D5 | PD071, 10 nmol/kg | 1.34 | 47.17 |
| D6 | PD071, 30 nmol/kg | -4.77 | 36.81 |
| D7 | PD071, 100 nmol/kg | -7.54 | 30.54 |

It could be seen from experimental results that, compared with the PD065 (Cagrilintide), the polypeptide derivative molecule in the present disclosure had better weight reduction effect under the same conditions.

### Example 7. Study on fatty acid side chain of derivatives

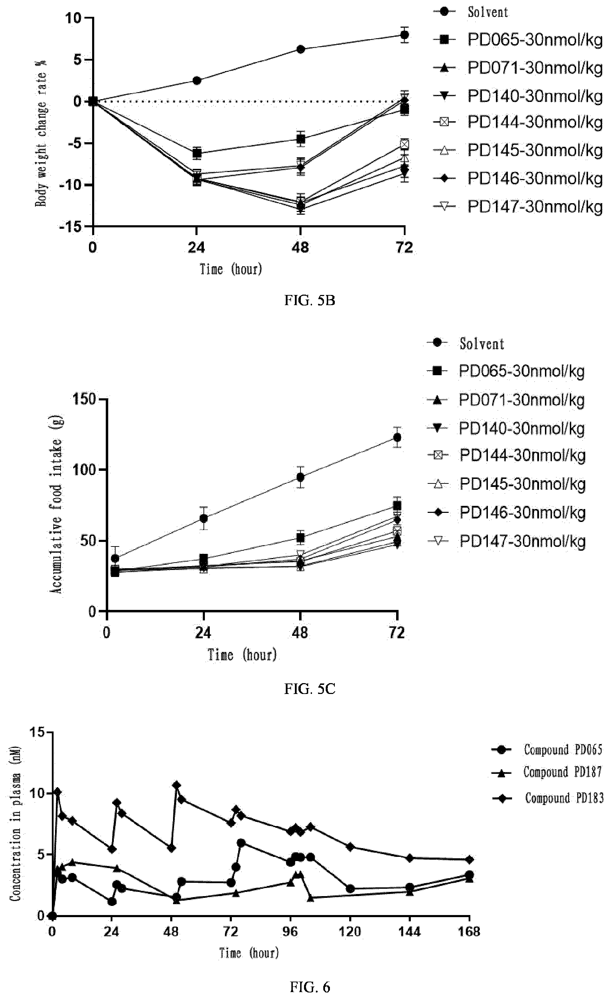

SD rats obtained by the same method as in Example 5 were used in this experimental study. There were an experimental group E: for testing weight reduction effects of the compounds PD065 and PD187, and an experimental group F: for testing the compounds PD065, PD071, PD140, PD144, PD145, PD146 and PD147, with 5 to 8 rats in each group of the derivative test, and the rats in each group were subcutaneously administered with a derivative or a solvent by a dosage of 30 nmol/kg. The administration time of each group was recorded. After administration, the rats were put back into their cages in which food and water available for the rats were placed. Food consumption and weight changes of the rats were recorded every 24 hours by online recording or manual recording. Results of the experimental group E were as shown in FIG. 5A, and results of the experimental group F were as shown in FIG. 5B and FIG. 5C.

Results of the animal experiment showed that, when an N-terminal was R (PD140), the activity was better than that of the PD065.

In order to further evaluate the effect of the fatty acid side chain on polypeptide activity, the PD071 (SEQ ID NO. 3) was used as a peptide backbone, and results showed that, when linkers of different fatty acids, such as 2AEEA+C20 diacid (PD187), αAsp+C20 diacid (PD144), βAsp+C20 diacid (PD145), Trx+C20 diacid (PD146) and Inp+C20 diacid (PD147) were used, the activity of the polypeptides was better than that of the PD065.

### Example 8. Preparation of polypeptide derivative tablet containing fatty acid side chain

A polypeptide derivative containing a fatty acid side chain was prepared into an oral tablet, and the tablet contained the same content of oral delivery agent PNAC. A preparation method of the PNAC was as follows.

N-[8-(2-hydroxybenzoyl)amino]octanoic acid (NAC) was prepared according to the method in Example 1 of the international patent application WO2008/028859. Isopropanol (22070.0 ml, 4.0 vol) was added into a 50 L reaction kettle, started to stir, and added with the NAC (5518 g, 1.0 eq). The system was heated to 50°C, and the prepared 50% potassium hydroxide solution (1304.0 g, 1.0 eq) was dropwise added into the system. After dropwise adding, the system turned into a clear and transparent yellow solution, and reacted under thermal insulation at 50°C for 1 hour. The reaction solution was concentrated in batches at 40°C, and a crude product was light orange yellow in color.

The crude product was added with isopropanol (19310.0 ml, 3.5 vol) in batches for pulping for 1 hour. The system was filtered, and a filter cake was eluted with isopropanol (2760.0 ml, 0.5 vol). The filter cake was transferred to a vacuum drying oven to be dried under a nitrogen cylinder pressure at 60°C for 16 hours, and then transferred to the vacuum drying oven again to be dried at 100°C for 24 hours. After drying, a total of 4.52 kg of product was obtained with a yield of 72.8%, which was a white powdery solid, that was, the PNAC.

Contents of main ingredients of the polypeptide derivative tablet containing the fatty acid side chain were as shown in Table 13:

**Table 13: Example of polypeptide derivative tablet prepared in the present application**

| Composition | API (polypeptide) Mass (mg/ tablet) | Delivery agent (PNAC) Mass (mg/ tablet) | Lubricant (such as magnesium stearate) Mass (mg/ tablet) | Total weight Mass (mg/ tablet) |
|---|---|---|---|---|
| 1 | 7 | 300 | 8 | 315 |

A preparation method of the polypeptide derivative tablet containing the fatty acid side chain was as follows: the polypeptide derivative and the PNAC were sieved, evenly mixed with excipients and directly tabletted.

### Example 9. Study on pharmacokinetical (PK) characteristics of polypeptide derivative tablet containing fatty acid side chain

Male Beagle dogs (9 kg to 12 kg) aged 10 months to 15 months were orally administrated with oral tablets prepared in Example 4 once a day for 5 consecutive days, and the dosages of the polypeptide derivatives (i.e. PD065, PD187 and PD183) containing the fatty acid side chains were respectively 7 mg/dog (n=5). The date of the first oral administration was recorded as Day 1, and the date of the last oral administration was recorded as Day 5. On the Day 1, whole blood of the animals before administration (-10 minutes) and at the 2^{nd} hour, the 4^{th} hour and the 8^{th} hour after administration was collected and plasma was prepared (with heparin sodium serving as an anticoagulant); on the Day 2 to the Day 4, whole blood of the animals before administration (-10 minutes) and at the 2^{nd} hour and the 4^{th} hour after administration was collected and plasma was prepared (with heparin sodium serving as an anticoagulant); and on the Day 5, whole blood of the animals before administration (-10 minutes) and at the 2^{nd} hour, the 4^{th} hour, the 8^{th} hour, 24^{th} hour, 48^{th} hour and 72^{nd} hour after administration was collected and plasma was prepared (with heparin sodium serving as an anticoagulant). The plasma was stored at -80°C and used for subsequent analysis. A drug content in the plasma was analyzed by LC-MS/MS (Waters ACQUITY I Class Premier UPLC tandem with Sciex 6500+QQQ). Experimental data were drawn by GraphPad Prism9.3.1. After the three polypeptide derivative tablets were administrated respectively, results of plasma concentrations of the three polypeptide derivatives (i.e. PD065, PD187 and PD183) measured at the time points respectively were as shown in FIG. 6.

Charge-to-mass ratio was a ratio of a charge of charged particles to a mass of the charged particles. A charge-to-mass ratio of ion pairs used in the PD065 was 1102.9/1074.5, a charge-to-mass ratio of ion pairs used in the PD187 was 1153/1120.9, and a charge-to-mass ratio of ion pairs used in the PD183 was 945.6/919.8.

It could be seen from the results as shown in FIG. 6 that: although the compounds PD187 and PD183 both had better weight reduction effect, in the same delivery environment, compared with the compound PD065-contained tablet group, the oral availability was not improved; and surprisingly, the compound PD183 molecule showed a special synergistic effect in the tablets of the PNAC, showing a very high oral bioavailability.

Although the examples of the present disclosure are described above, the present disclosure is not limited to the above specific examples and application fields, and the above specific examples are only illustrative and instructive, and are not restrictive. Under the inspiration of the specification, those of ordinary skills in the art may also make many forms without departing from the scope of protection of the claims of the present disclosure, all of which belong to the protection of the present disclosure.

## Claims

1. An amylin polypeptide analog or a derivative thereof, or a pharmaceutically acceptable salt thereof, comprising an amino acid sequence as shown in formula (I):
X₁CNTATCATQRLAX₁₄FLX₁₇HX₁₉X₂₀X₂₁X₂₂X₂₃X₂₄X₂₅ILX₂₈X₂₉TNVGSX₃₅TX₃₇ formula (I),
wherein,
X₁ is selected from K, D, Dab, Dap, E, H, Har, Orn or R, or deleted,
X₁₄ is selected from N, Aad, Dab, Dap, E or D,
X₁₇ is selected from V, Orn or R,
X₁₉ is selected from S or K,
X₂₀ is selected from S or K,
X₂₁ is selected from N or K,
X₂₂ is selected from N or K,
X₂₃ is selected from F, L or K,
X₂₄ is selected from G, R, H, P, D, N, S, K, αK, Dap, Dab, Orn, Cit, Sar, Trx or AOC,
X₂₅ is selected from A, K or P,
X₂₈ is selected from S or P,
X₂₉ is selected from S or P,
X₃₅ is selected from N or G, and
X₃₇ is selected from Y, 3-Am-Pro, Aze, cis-Hyp, P, Pip, αMePro or trans-Hyp.

2. The polypeptide analog or the derivative thereof, or the pharmaceutically acceptable salt thereof according to claim 1, comprising an amino acid sequence as shown in formula (II):
X₁CNTATCATQRLAX₁₄FLX₁₇HSSNNX₂₃X₂₄X₂₅ILX₂₈X₂₉TNVGSNTX₃₇ formula (II),
wherein,
X₁ is selected from K or R,
X₁₄ is selected from N or D,
X₁₇ is selected from V or R,
X₂₃ is selected from F or L,
X₂₄ is selected from G, R or K,
X₂₅ is selected from A or P,
X₂₈ is selected from S or P,
X₂₉ is selected from S or P, and
X₃₇ is selected from Y, P or trans-Hyp.

3. The polypeptide analog or the derivative thereof, or the pharmaceutically acceptable salt thereof according to claim 2, wherein the X₁₄ is D, the X₁₇ is R, the X₂₃ is L, the X₂₅ is P, the X₂₈ is P, the X₂₉ is P, and the X₃₇ is P or trans-Hyp.

4. The polypeptide analog or the derivative thereof, or the pharmaceutically acceptable salt thereof according to claim 3, wherein the X₂₄ is K or R; and preferably, the X₁ is K.

5. The polypeptide analog or the derivative thereof, or the pharmaceutically acceptable salt thereof according to claim 1 or 2, comprising an amino acid sequence selected from SEQ ID NO. 2 to 104.

6. The polypeptide analog or the derivative thereof, or the pharmaceutically acceptable salt thereof according to any one of claims 1 to 5, wherein the derivative or the pharmaceutically acceptable salt thereof comprises 1 to 10 amino acid modifications.

7. The polypeptide analog or the derivative thereof, or the pharmaceutically acceptable salt thereof according to claim 6, wherein the modification is located on a Epsilon amino group of K that at 1^{st} position.

8. The polypeptide analog or the derivative thereof, or the pharmaceutically acceptable salt thereof according to claim 6 or 7, wherein the amino acid modification is to introduce a functional group capable of being used for coupling into an amino acid side chain.

9. The polypeptide analog or the derivative thereof, or the pharmaceutically acceptable salt thereof according to claim 8, wherein the functional group capable of being used for coupling comprises an extension part and a linker linking the extension part to a modified amino acid.

10. The polypeptide analog or the derivative thereof, or the pharmaceutically acceptable salt thereof according to claim 9, wherein the extension part is selected from 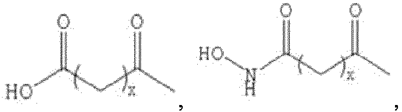 or 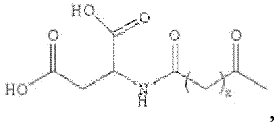 wherein x is any integer from 4 to 38;
preferably,
the extension part is selected from:
HOOC(CH₂)₁₄CO-, HOOC(CH₂)₁₅CO-, HOOC(CH₂)₁₆CO-, HOOC(CH₂)₁₇CO-, HOOC(CH₂)₁₈CO-, HOOC(CH₂)₁₉CO-, HOOC(CH₂)₂₀CO-, HOOC(CH₂)₂₁CO- and HOOC(CH₂)₂₂CO-.

11. The polypeptide analog or the derivative thereof, or the pharmaceutically acceptable salt thereof according to claim 9 or 10, wherein the linker is selected from: 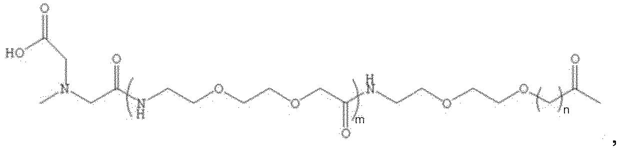 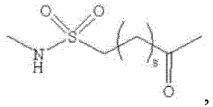 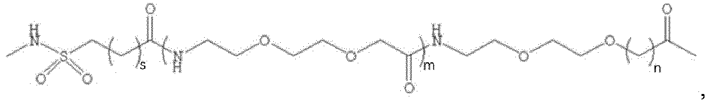 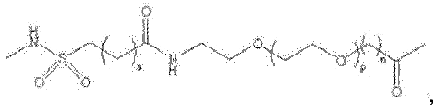 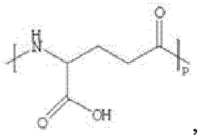 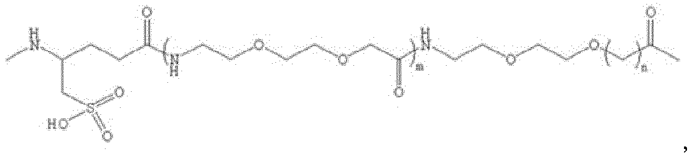 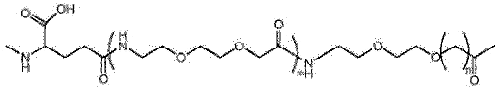 or 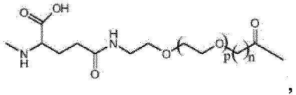 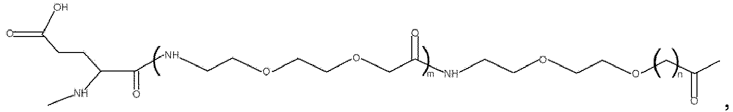 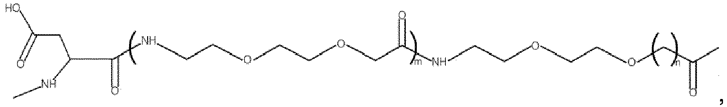 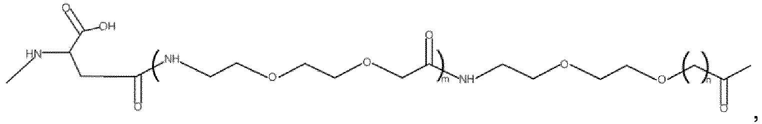 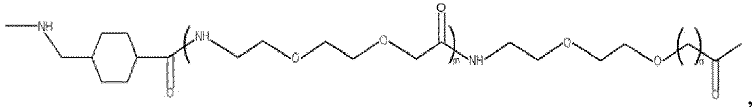 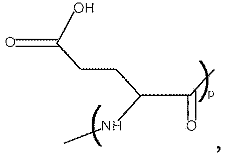 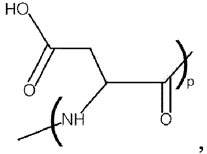 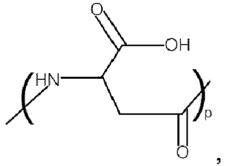 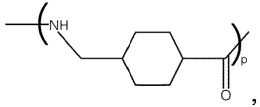 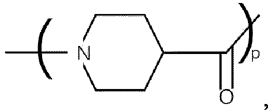 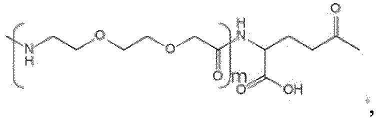 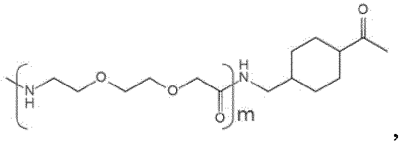 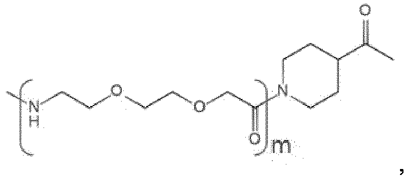 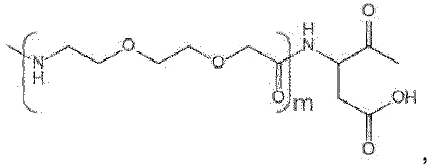 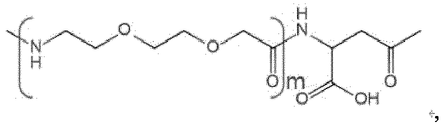 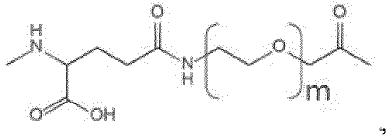 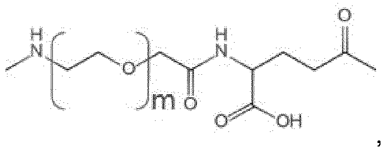 wherein m is 0, 1, 2 or 3; n is 1, 2 or 3; s is any integer from 0 to 6; and p is any integer from 1 to 8;
preferably, the linker is selected from: 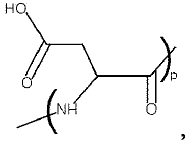 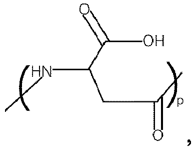 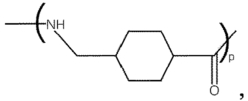 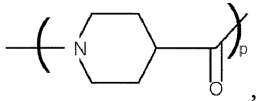 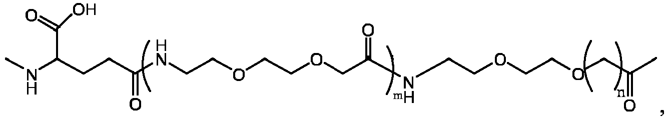 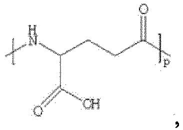 wherein m is 1, n is 1 or 2, and p is any integer from 1 to 5.

12. The polypeptide analog or the derivative thereof, or the pharmaceutically acceptable salt thereof according to claim 11, wherein, the derivative or the pharmaceutically acceptable salt thereof is selected from PD069, PD070, PD071, PD072, PD140, PD158, PD160, PD161, PD144, PD145, PD146, PD147 or PD187.

13. The polypeptide analog or the derivative thereof, or the pharmaceutically acceptable salt thereof according to claim 12, wherein, the derivative or the pharmaceutically acceptable salt thereof is

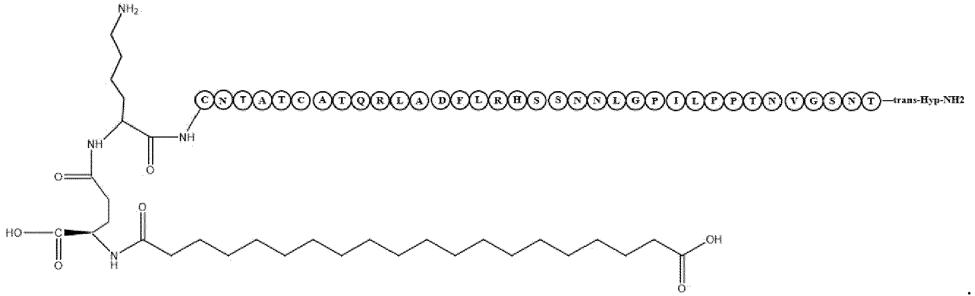

14. The polypeptide analog or the derivative thereof, or the pharmaceutically acceptable salt thereof according to claim 12, wherein, the derivative or the pharmaceutically acceptable salt thereof is

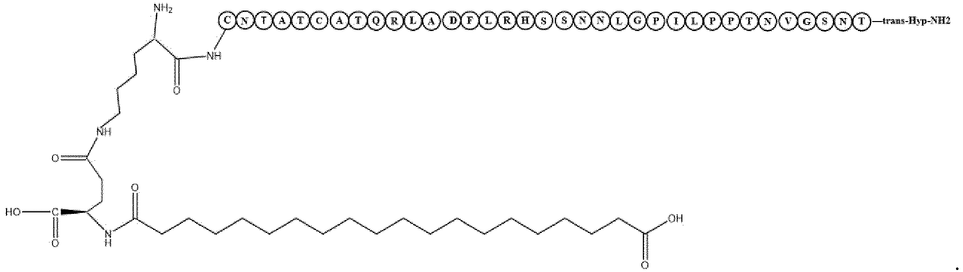

15. The polypeptide analog or the derivative thereof, or the pharmaceutically acceptable salt thereof according to claim 12, wherein, the derivative or the pharmaceutically acceptable salt thereof is

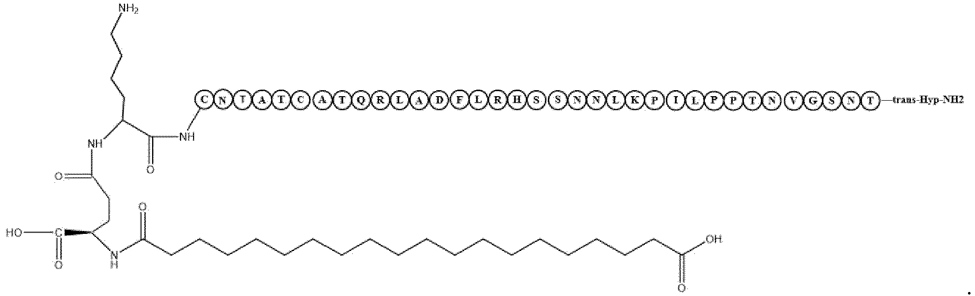

16. The polypeptide analog or the derivative thereof, or the pharmaceutically acceptable salt thereof according to claim 12, wherein, the derivative or the pharmaceutically acceptable salt thereof is

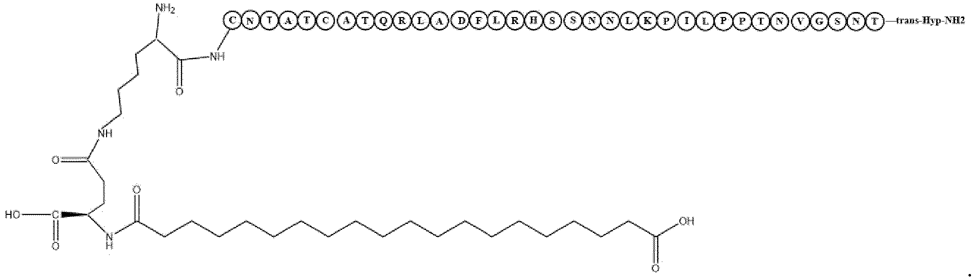

17. A preparation method of the polypeptide analog or the derivative thereof, or the pharmaceutically acceptable salt thereof according to any one of claims 1 to 16.

18. Use of the polypeptide analog or the derivative thereof, or the pharmaceutically acceptable salt thereof according to any one of claims 1 to 16 in preparation of a medicament for preventing or treating a disease.

19. The use according to claim 18, wherein the disease is an amylin receptor-related disease.

20. The use according to claim 19, wherein the amylin receptor-related disease is selected from fat metabolism disorder, blood glucose metabolism disorder, a cardiovascular disease, and a brain, mental or nervous system disease.

21. A method for treating or/and preventing a disease, wherein the method comprises administering the polypeptide analog or the derivative thereof, or the pharmaceutically acceptable salt thereof according to any one of claims 1 to 16, a medicament or a pharmaceutical composition thereof to a subject in need.

22. The method according to claim 21, wherein the disease is selected from fat metabolism disorder, blood glucose metabolism disorder, a cardiovascular disease, and a brain, mental or nervous system disease.

23. A pharmaceutical composition, comprising the polypeptide analog or the derivative thereof, or the pharmaceutically acceptable salt thereof according to any one of claims 1 to 16.

24. The pharmaceutical composition according to claim 23, further comprising one or more oral delivery agents; preferably, the oral delivery agent is N-[8-(2-hydroxybenzoyl)amino]octanoic acid (NAC) salt; and further preferably, the oral delivery agent is PNAC.

25. The pharmaceutical composition according to claim 23 or 24, further comprising another or more pharmacologically active substances.

26. The pharmaceutical composition according to claim 25, wherein the pharmacologically active substance is selected from a body weight regulator, an anti-obesity agent, a lipid metabolism regulator, a blood glucose regulator, a hypertension therapeutic agent, a cardiovascular regulator, a brain system regulator, a mental system regulator or a nervous system regulator.
